# EUROPÄISCHE PATENTSCHRIFT

(11) **EP 3 630 945 B1**
(45) Veröffentlichungstag und Bekanntmachung des Hinweises auf die Patenterteilung: **02.08.2023**
(21) Anmeldenummer: 18729041.6
(22) Anmeldetag: 28.05.2018
(51) Int. Cl.: C12N 5/00

(54) **ZELLKULTUREN ENTHALTEND POLY(OXAZOLIN)-STABILISATOREN UND VERWENDUNG VON POLY(OXAZOLINEN) ZUR STABILISIERUNG VON ZELLKULTUREN**
CELL CULTURES COMPRISING POLY(OXAZOLINE) STABILIZERS AND USE OF POLY(OXAZOLINES) FOR STABILIZING CELL CULTURES
CULTURES CELLULAIRES CONTENANT DES POLY(OXAZOLINE)S COMME STABILISANTS ET UTILISATION DE POLY(OXAZOLINES) POUR LA STABILISATION DE CULTURES CELLULAIRES

(30) Priorität: 26.05.2017 DE 102017005048
(43) Veröffentlichungstag der Anmeldung: 08.04.2020
(73) Patentinhaber: Sartorius Xell GmbH, 33758 Schloß Holte-Stukenbrock (DE)
(72) Erfinder: LEISKE, Meike, Nicole, 27749 Delmenhorst (DE); TRAEGER, Anja, 95367 Trebgast (DE); SCHUBERT, Ulrich, Sigmar, 07743 Jena (DE)
(74) Vertreter: Ackermann, Joachim
(86) Internationale Anmeldenummer: PCT/EP2018/000275
(87) Internationale Veröffentlichungsnummer: WO 2018/215094

(56) Entgegenhaltungen:
- TIM R DARGAVILLE ET AL: "Poly(2-oxazoline) hydrogels as next generation three-dimensional cell supports", CELL ADHESION AND MIGRATION, Bd. 8, Nr. 2, 18. Februar 2014 (2014-02-18), Seiten 88-93, XP55489029, US ISSN: 1933-6918, DOI: 10.4161/cam.28205
- TACEY X. VIEGAS ET AL: "Polyoxazoline: Chemistry, Properties, and Applications in Drug Delivery", BIOCONJUGATE CHEMISTRY, Bd. 22, Nr. 5, 18. Mai 2011 (2011-05-18), Seiten 976-986, XP55082338, ISSN: 1043-1802, DOI: 10.1021/bc200049d
- Anonymous: "Poly(2-oxazoline)s (POx) in Biomedical Applications | Sigma-Aldrich", , 1. Januar 2016 (2016-01-01), XP55489018, Gefunden im Internet: URL:https://www.sigmaaldrich.com/technical -documents/articles/material-matters/poly- 2-oxazoline-s-pox-in-biomedical-applicatio ns.html [gefunden am 2018-06-29]
- CHANG DAVID ET AL: "Investigation of interfacial properties of pure and mixed poloxamers for surfactant-mediated shear protection of mammalian cells", COLLOIDS AND SURFACES. B, BIOINTERFACES, ELSEVIER, AMSTERDAM, NL, Bd. 156, 16. Mai 2017 (2017-05-16), Seiten 358-365, XP085082047, ISSN: 0927-7765, DOI: 10.1016/J.COLSURFB.2017.05.040
- T. THARMALINGAM ET AL: "Pluronic Enhances the Robustness and Reduces the Cell Attachment of Mammalian Cells", MOLECULAR BIOTECHNOLOGY, Bd. 39, Nr. 2, 8. März 2008 (2008-03-08), Seiten 167-177, XP55129642, ISSN: 1073-6085, DOI: 10.1007/s12033-008-9045-8
- TAE GON KIM ET AL: "Poly-[gamma]-glutamic acid enhances the quality of recombant erythropoietin produced by CHO cells", BIOTECHNOLOGY & BIOTECHNOLOGICAL EQUIPMENT, Bd. 28, Nr. 2, 4. März 2014 (2014-03-04), Seiten 350-354, XP55489035, BG ISSN: 1310-2818, DOI: 10.1080/13102818.2014.901675
- SHAH RUSHITA ET AL: "In vitro study of partially hydrolyzed poly(2-ethyl-2-oxazolines) as materials for biomedical applications", JOURNAL OF MATERIALS SCIENCE: MATERIALS IN MEDICINE, SPRINGER NEW YORK LLC, UNITED STATES, Bd. 26, Nr. 4, 18. März 2015 (2015-03-18), Seiten 1-12, XP035491112, ISSN: 0957-4530, DOI: 10.1007/S10856-015-5485-4 [gefunden am 2015-03-18] in der Anmeldung erwähnt
- S Klausing ET AL: "Surfactants in cell culture media: Impact on HEK and CHO cells in cultivation and transfection", , 17. Mai 2017 (2017-05-17), XP055489236, Gefunden im Internet: URL:https://www.xell.ag/shop/media/pdf/da/ 9b/52/Poster_Xell_ESACT2017_Surfactants.pd f [gefunden am 2018-06-29]
- S Klausing: "Surfactants in cell culture media: Impact on HEK and CHO cells in cultivation and transfection; PO153", , 17. Mai 2017 (2017-05-17), XP055489238, Gefunden im Internet: URL:http://www.esact2017.com/wp-content/up loads/2015/05/ESACT-2017-Abstracts2.pdf [gefunden am 2018-06-29]

## Beschreibung

Die Erfindung betrifft das Gebiet der Kultivierung von Zellen in einem Zellkulturmedium und die Verwendung von Poly(oxazolin)en zur Stabilisierung von Zellkulturen.

Beschrieben werden Zellkulturen enthaltend in einem turbulent bewegten Zellkulturmedium ein oder mehrere wasserlösliche Poly(oxazolin)e. Das wasserlösliche Poly-(oxazolin) wirkt als Stabilisator für die Zellen und verringert den mechanischen Stress, der durch das Bewegen des Zellkulturmediums auf die Zellen ausgeübt wird. Dieses führt zu einer gegenüber dem unstabilisierten Zustand verbesserten Überlebensrate der Zellen.

Die Kultivierung von Zellen in Zellkulturmedien außerhalb eines Organismus ist bekannt. Zellkulturen finden breite Verwendung in Forschung, Entwicklung und Produktion.

Bei der Kultivierung von Zellen werden Suspensionen von Zellen in einem Zellkulturmedium oder von auf Oberflächen anhaftenden Zellen in einem Zellkulturmedium wachsen gelassen.

Dabei ist man bestrebt, eine möglichst hohe Volumendichte an Zellen im Zellkulturmedium oder auf der Oberfläche zu erreichen. Dieses verspricht eine erhöhte Ausbeute an Zellen und an gewünschten Stoffwechselprodukten, kann aber auch zu Problemen führen. Infolge der hohen Zellaktivität kann es zu einer erschwerten Versorgung der Zellen mit lebensnotwendigen Agenzien, beispielsweise mit Nährstoffen oder mit Sauerstoff, kommen. Dieses versucht man unter anderem durch starkes Rühren des Nährmediums zu verhindern. Dabei entstehen zwangsläufig im Zellkulturmedium oder in der Nähe der Oberflächen Turbulenzen, welche wiederum die Zellen mechanisch belasten und damit schädigen können.

Aus dem Stand der Technik ist der Zusatz ausgewählter wasserlöslicher Polymerer zu_Zellkulturmedien bereits bekannt und erprobt. Typische Additive in diesem Zusammenhang sind Polyethylenglykol-polypropylenglykol-Blockcopolymere, die z.B. als Kolliphor^{®} P188 oder als Pluronic^{®} F68 erhältlich sind. Durch den Einsatz dieser Stabilisatoren lässt sich die Belastung der Zellen verringern.

S. Lueck *et al.* und M. Platen *et al.* synthetisierten hydrogelbasierte "Microbeads" ausgehend von Methacrylatmonomeren, die mit Poly(oxazolin)en quervernetzt wurden. Diese Polymere dienen als bioabbaubare Hydrogeltransportsysteme für Stammzellkulturen. Hierbei wurden wasserunlösliche Matritzen mittels Polymere aufgebaut, um die wenigen Stammzellen schonend und "artgerecht" zu kultivieren. Die Polymere liegen also nicht in Lösung vor. Dies unterscheidet sich damit grundlegend von einem Ansatz, bei dem Polymere zum Zellkulturmedium gegeben werden und diese Polymere damit in diesem Medium gelöst vorliegen und für eine Kultivierung von Zellen unter Stress eingesetzt werden. Die Zellen werden dabei nicht fest in eine Matrize eingebettet (Biomaterials, 2016, 79, 1-14, S. Lueck, R. Scubel, J. Rueb, D. Hahn, E. Mathieu, H. Zimmermann, D. Schwarnweber, C. Werner, S. Pautot, R. Jordan, Tailored and biodegradable poly(2-oxazoline) microbeads as 3D matrices for stem cell culture in regenerative therapies; Biomacromolecules, 2015, 16, 1516-1524, M. Platen, E. Mathieu, S. Lueck, R. Schubel, R. Jordan, S. Pautot, Poly(2-oxazoline)-based microgel particles for neuronal cell culture).

A. Dworak *et al.*, A. Utrata-Wesolek *et al.* und P. I. Haris *et al.* funktionalisierten Oberflächen, indem die reaktive Spezies der Ringöffnungspolymerisation auf eine Amin-funktionalisierte Oberfläche gebracht wurde, um die Polymere so kovalent an die Oberfläche zu binden, während die Polymerisation terminiert wird. Diese funktionalisierten Oberflächen wurden dann in Fibroblasten-Zellkulturen genutzt, um diese reversibel anzuheften. Y. Chen *et al.* verglichen diesbezüglich die Eigenschaften von Poly(2-methy-2-oxazolin-g-L-lysin) mit Poly(ethylenglycol-g-lysin) und gehen zusätzlich auf die Serumstabilität der Polymere ein. Sie fanden heraus, dass das Poly(2-oxazolin) basierte System eine höhere Serumstabilität aufweist als das Poly(ethylenglycol) basierte System. A. Tait *et al.* nutzten für eine Oberflächenbeschichtung nach einem ähnlichen Prinzip Poly(2-methyl-2-oxazolin), Poly(2-ethyl-2-oxazolin), Poly(2-propyl-2-oxazolin) und Poly(2-butyl-2-oxazolin) und verwendeten die so modifizierten Oberflächen zur Züchtung von Geweben. Bei diesen Ansätzen wurden also jeweils immobilisierte Polymere eingesetzt und es wurden sowohl hydrophile also auch hydrophobe Polyoxazoline eingesetzt (J. Mater Sci. Mater. Med., 2014, 25, 1149-1163, A. Dworak, A. Utrata-Wesolek, N. Oleszko, W. Walach, B. Trzebicka, J. Aniol, A. L. Sieron, A. Klama-Baryla, M. Kawecki. Poly(2-substituted-2-oxazoline) surfaces for dermal fibroblasts adhesion and detachment; EP 2 574 664 A1, A. Utrata-Wesolek, W. Walach, N. Oleszko, A. Dworak, B. Trzebicka, A. Kowalczuk, J. Aniol, M. Lesiak, A. Sitkowska, A. L. Sieron, M. Kawecki, J. Glik, A. Klama-Baryla, M. Nowak, Method for preparation a thermosensitive coating on-a substrate, the substrate with a thermosensitive coating and its application; BioMedical Materials and Engineering, 2004, 14, 419-425. P. I Haris, M. Dahm, J. Ruehe, B. Berchthold, D. Pruefer, O. Prucker, B.-J. Chang, A. Wallrath, H. Oelert, Ultrathin polymer monolayers for promotion of cell growth on bioprosthetic materials - Evolution of a new concept to improve long term performance of biologic heart vales; Biointerphases, 2014, 9, Y. Chen, B. Pidhatika, T. von Erlach, R. Konradi, M. Textor, H. Hall, T. Luhmann doi: 10.1116/14878461; Biomaterials, 2015, 61, 26-32, A. Tait, A. L. Fisher, T. Hartland, D. Smart, P. Glynne-Jones, M. Hill, E. J. Swindle, M. Grossel, D. E. Davies, Biocompatibility of poly(2-alkyl-2-oxazoline) brush surfaces for adherent lung cell lines).

R. Himmelreich, S. Werner und M. N. Leiske *et al.* nutzen Poly(2-oxazolin)e, um Nukleinsäuren aus biologischen Proben aufzureinigen. Dazu wurden in diese zusätzliche funktionelle Gruppen, wie Amine eingeführt. Die genannten Quellen nutzten funktionalisierte Poly(2-oxazoline) zur Aufreinigung von Nukleinsäuren und nicht als Zellkulturzusatz (EP 2 163 621 A1 entsprechend WO 2010/026167 A1, R. Himmelreich, S. Werner, Method and reagents for isolating and purifying nucleic acids from biological samples or from biochemical reactions by lysis, adhesion, washing, and elution; Adv. Func. Mater., 2015, 25, 2458-2466, M. N. Leiske, M. Hartlieb, C. Paulenz, D. Pretzel, M. Hentschel, C. Englert, M. Gottschaldt, U. S. Schubert, Lab in a tube: Purification, amplification, and detection of DNA using poly(2-oxazoline) multilayers).

Tim R. Darqaville et al. in Cell Adhesion and Migration, Bd. 8, Nr. 2, S. 88-93 beschreiben Poly(2-oxyzolin)-Hydroqele, die als Gerüst für die 3D Kultivierung von Zellen dienen sollen. Bei den Hydrogelen handelt es sich um hochvernetzte Strukturen, die ein dreidimensionales Netzwerk ausbilden. Diese Hydrogele sind nicht wasserlöslich bzw. sind als makroskopische Strukturen in Wasser gelöst.

Tacev S. Viegas et al. in Bioconjugate Chemistry, Bd. 22, Nr. 5, S. 976-986 (2011) offenbaren Chemie, Eigenschaften und Anwendungen von Poly-(oxazolinen) für den Einsatz in der Pharmakotherapie. In diesem Dokument wird unter anderem die Verträglichkeit von Erythrozyten mit PEOZ (= Polyethyloxazolin) bzw. mit Polyethylenglykol (PEG) untersucht. Bei dem dort beschriebenen Testsystem handelt es sich aber nicht um eine Zellkultur, da dem in dort gewählten Ansatz ein Zellkulturmedium fehlt.

R. Luxenhofer et al. berichten in Material Matters, 2016 über Poly(2-oxazoline) (PO)ₓ in biomedizinischen Anwendungen. In diesem Dokument wird kein Zellkulturadditiv beschrieben, sondern es werden Zellkulturen genutzt, um die Funktion von Polymeren zu untersuchen.

David Chang et al. berichten in Colloids and Surfaces. B, Biointerfaces, Elsevier, Bd. 156, 16. Mai 2017, S. 358-365 über eine Untersuchung der Grenzflächeneigenschaften von reinen und gemischten Poloxameren zum Schutz von Säugetierzellen gegenüber Scherbeanspruchung.

T. Tharmalingam et al. berichten in Molecular Biotechnology (2008) 39: 167-177 über den Einsatz von Pluronic^{®} F-68 zum Schutz von Säuetierzellen in serum-freien Medien gegenüber Scherbeanspruchung.

Tae Gon Kom et al. beschreiben in Biotechnology & Biological Equipment, 2014, Bd, 28, Nr. 2, S. 350-354 den Einsatz von Poly-gamma-glutaminsäure zur Qualitätsverbesserung von rekombinantem Erythropoietin, das durch CHO-Zellen erzeugt wird.

Rushita Shah et al. berichten in J. of Materials Science: Materials in Medicine (2015), Springer ,Bd. 26, S. 1-12 über eine in-vitro Studie von teilweise hydrolysierten Poly(-2-ethyl-2-oxazolinenen) für den Einsatz in biomedizinischen Anwendungen. Diese Arbeit offenbart keine bewegten Zellkulturmedien, so dass keine Anwendung von stabilisierenden Tensiden erforderlich ist.

Versuche mit unterschiedlichen Tensiden in Zellkulturmedien werden von S. Klausing et al. in https://www.xell.ag-/shop/media/pdf/da/9b/52/Poster Xell_ESACT2017Surfactants.pdf sowie in http://esact2017.com/wpcontent/uploads/2015/05/ESACT-2017-Abstracts2.pdf beschrieben.

Die aus dem im Stand der Technik bekannten polymerbasierten Systeme, welche Poly(2-oxazolin)e anwenden, setzten diese immobilisiert in einem Kultivierungsgefäß, jedoch nicht als Mediumzusatz bei der Zellkultur ein. Dieses erfolgt zur Verhinderung der Adhäsion / des Wachstums der Zellen an einer Oberfläche. Zu diesem Zweck werden die Oberflächen mit biokompatiblen Polymeren beschichtet, welche auf der Oberfläche immobilisiert sind.

Bekannte polymerbasierte gelöste Zusätze in Zellkulturen sind die bereits weiter oben erwähnten hydrophilen Copolymere Pluronic^{®} F68 oder Kolliphor^{®} P188.

Es wurde nun überraschend gefunden, dass bei Verwendung von ausgewählten Poly(oxazolin)en als Stabilisatoren in Zellkulturmedien bei der Kultivierung der Zellen bei gleicher Konzentration ein erhöhter positiver Beitrag zur Verbesserung der Überlebensrate der Zellen resultiert. Der Einsatz dieser ausgewählten Poly(oxazolin)e als Stabilisatoren in Zellkulturmedien ist bisher nicht beschrieben worden.

Teilweise hydrolysierte Poly(2-ethyl-2-oxazoline) wurden als Zusätze zu Kulturen von 3T3 Fibroblasten, βTC3 Pankreaszellen oder P388.D1 Makrophagen eingesetzt (J. Mater Sci: Mater Med 26:157, S. 1-12, 2015, R. Shah, Z. Kronekova, A. Zahoranovä, L. Roller, N. Saha, P. Saha, J.Kronek, In vitro study of partially hydrolyzed poly(2-ethyl-2-oxazolines) as materials for biomedical applications). In dieser Arbeit wurde die Cytotoxizität von Polyoxazolin-Polyethylenimin-Copolymeren untersucht, da lineares Polyethylenimin aus Poly(2-ethyl-2-oxazolin) durch Hydrolyse hergestellt wird. Dabei wurden Poly(2-ethyl-2-oxazoline) als Vergleichssubstanzen eingesetzt. Zur Untersuchung der Cytotoxizität wurden die Zellen in Gegenwart von fötalem Rinderserum (FBS) oder von Pferdeserum (HS) kultiviert, sodann mit dem betreffenden Polymer und MTT Lösung versetzt und weitere 2 Stunden lang kultiviert. Danach wurden die Zellen isoliert und es wurde deren Absorbanz bei 595 nm als Maß für deren Lebenfähigkeit bestimmt.

In einer Reihe von biotechnologischen Anwendungen ist der Einsatz von Seren bei der Kultivierung von Zellen nicht gewünscht oder nicht möglich. Ein Grund dafür liegt neben ethischen Aspekten in der natürlich gegebenen Chargenschwankung und der daraus folgenden fehlenden Reproduzierbarkeit sowie potentiellen Kontaminationen und/oder Immunogenitäten von Seren, die zu Komplikationen bei Patienten führen können. Des Weiteren können Seren Verunreinigungen enthalten und Krankheiten übertragen. Daher werden bei der Zellkultivierung vermehrt serum-freie Kulturmedien, besser noch chemisch definierte Medien, angestrebt, in denen somit Komponenten mit definierteren und reproduzierbaren Eigenschaften vorliegen.

Die Aufgabe der vorliegenden Erfindung bestand in der Bereitstellung von Zellkulturen, die sich durch eine hervorragende Stabilität während und nach der Kultivierung und durch eine gegenüber dem unstabilisierten Zustand verbesserte Überlebensrate auszeichnen.

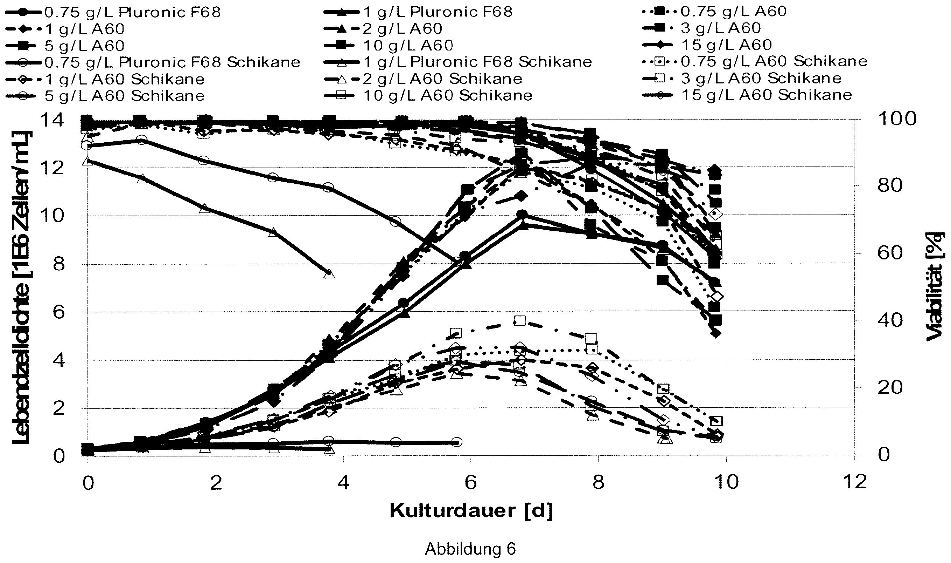

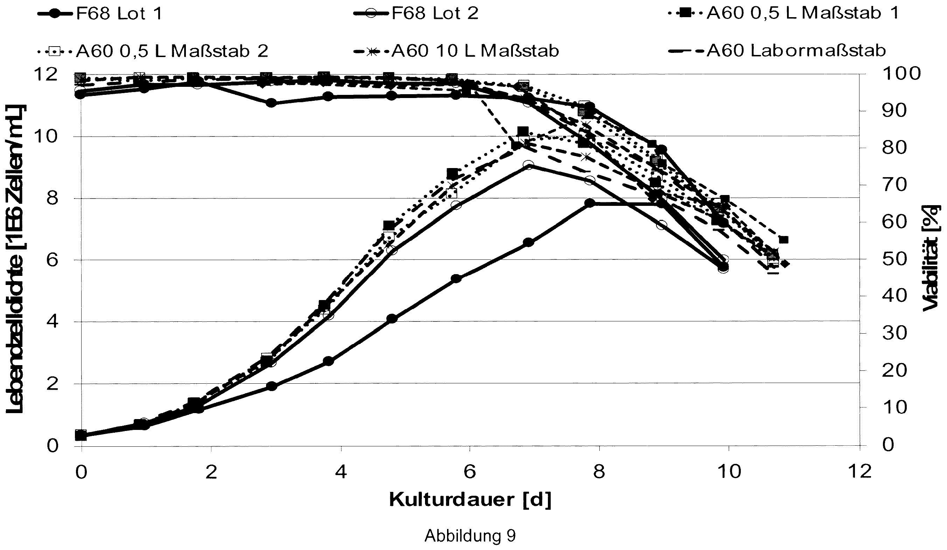

Das technische Problem wird durch die Bereitstellung von Zellkulturen, die in einem turbulent bewegten Zellkulturmedium ein oder mehrere Poly(oxazolin)e enhalten gelöst. Wie oben erwähnt ist aus dem Stand der Technik der Zusatz ausgewählter wasserlöslicher Polymere, wie beispielsweise Pluronic^{®} F68 bekannt. Wie bspw. in Abbildung 6 der Anmeldung gezeigt, sank die Viabilität einer Zellkultur enthaltend Pluronic^{®} F68 innerhalb von 3 bzw. 4 Tagen auf unter 60%. Ferner wurde eine sogenannte lot-to-lot Variabilität bei dem Zusatz Pluronic^{®} F68 gezeigt (Beispiel 5). Im Gegensatz dazu zeigten die Zellkulturen der vorliegenden Erfindung, die in einem Zellkulturmedium ein oder mehrere Poly(oxazolin)e enthalten, selbst unter hohen Scherbelastungen deutlich erhöhte maximale Zelldichten, erhöhte Viabilitäten und eine längere Kulturdauer gegenüber dem Zusatz Pluronic^{®} F68 (vgl. hierzu Abbildung 6 sowie die Tabellen 1, 2 und 3). Ferner konnte kein bedeutender Unterschied verschiedener getesteter Poly(oxazolin)e Lots untereinander in Zellkulturen erkannt werden (vgl. hierzu bspw. Abbildung 9 und Beispiel 5).

Somit betrifft die vorliegende Erfindung Zellkulturen, die ein oder mehrere wasserlösliche Poly(oxazolin)e im turbulent bewegten Zellkulturmedium enthalten. Bevorzugt betrifft die vorliegende Erfindung Zellkulturen, die ein oder mehrere wasserlösliche Poly(oxazolin)e im turbulent bewegten Zellkulturmedium enthalten, mit der Maßgabe, dass das Zellkulturmedium im Wesentlichen kein Serum enthält. Folglich betrifft die vorliegende Erfindung vorzugsweise Zellkulturen, die ein oder mehrere wasserlösliche Poly(oxazolin)e in einem turbulent bewegten Zellkulturmedium enthalten, mit der Maßgabe, dass das eingesetzte Zellkulturmedium im Wesentlichen serum-frei ist.

Bevorzugte erfindungsgemäße Zellkulturen sind dadurch gekennzeichnet, dass die Zellkulturen und insbesondere das Zellkulturmedium frei von tierischen Bestandteilen sind. Ferner betrifft die Erfindung Zellkulturen, die dadurch gekennzeichet sind, dass das eingesetzte Zellkulturmedium protein-frei ist. Besonders bevorzugt sind im Rahmen der vorliegenden Erfindung Zellkulturen, enthaltend ein Zellkulturmedium, das serum-frei und/oder protein-frei ist.

Weitere bevorzugte erfindungsgemäße Zellkulturen sind solche, die Zellkulturmedien mit nur definierteren chemischen und/oder biotechnologischen Bestandteilen enthalten. In anderen Worten sind bevorzugte Zellkulturen solche, die dadurch gekennzeichnet sind, dass das Zellkulturmedium ein chemisch definiertes Medium ist.

Unter "Zellen" sind im Rahmen der vorliegenden Beschreibung kleinste lebende Einheiten von Organismen zu verstehen. Dabei kann es sich um Zellen von Ein- oder Mehrzellern handeln, welche von Prokaryonten oder von Eukaryoten stammen können. Bei den Zellen kann es sich um Mikroorganismen, um einzelne Zellen oder um Gewebe handeln. Zellen können prokariontischen, pflanzlichen oder tierischen Ursprungs sein oder auch von Pilzen stammen. Vorzugsweise werden eukaryotische Zellen eingesetzt, insbesondere solche, die ursprünglich aus Gewebe isoliert wurden und dauerhaft kultiviert werden können, die also immortalisiert sind.

Unter "Zellkultur" werden im Rahmen der vorliegenden Beschreibung Kombinationen von Zellen und Zellkulturmedium bezeichnet, wobei die Zellen in dem Zellkulturmedium außerhalb des Organismus kultiviert werden. Dabei kommen Zelllinien zum Einsatz, also Zellen einer Gewebeart, die sich im Verlauf der Kultivierung fortpflanzen können. Es können sowohl immortalisierte (unsterbliche) Zelllinien als auch primäre Zellen (Primärkultur) kultiviert werden. Unter Primärkultur ist üblicherweise eine nicht immortalisierte Zellkultur zu verstehen, die direkt aus einem Gewebe gewonnen wurde.

Unter "Zellkulturmedium" oder "Nährmedium" sind im Rahmen der vorliegenden Beschreibung wässrige Systeme zu verstehen, die als Plattform für die Kultivierung von Zellen dienen. Diese Systeme enthalten alle benötigten Substanzen, die für das Wachstum und die Viabilität der Zellen erforderlich sind.

Unter "im Wesentlichen kein Serum enthaltende Zellkulturmedien" sind im Rahmen der vorliegenden Beschreibung Zellkulturmedien zu verstehen, die kein Serum oder nur geringen Mengen von bis zu 1 Gew. %, vorzugsweise von weniger als 0,1 Gew. %, bezogen auf das Zellkulturmedium an Serum enthalten.

Die Verwendung von Zellkulturmedien im Bereich der pharmazeutischen Industrie, beispielsweise zur Herstellung von Arzneimitteln, wie von aktiven rekombinanten Polypeptiden, erlaubt im Allgemeinen keine Verwendung von irgendeinem Material biologischen und/oder tierischen Ursprungs aufgrund von Sicherheits- und Kontaminationsproblemen. Daher ist das eingesetzte Zellkulturmedium gemäß der vorliegenden Erfindung vorzugsweise ein serum- und/oder protein-freies Medium.

Der Begriff "Proteine" bedeutet im Rahmen dieser Beschreibung Proteine aus mehr als 100 Aminosäuren. Demnach kann das Zellkulturmedium der vorliegenden Erfindung bspw. rekombinates Insulin (bestehend aus 51 Aminosäuren) enthalten.

Das Zellkulturmedium gemäß der vorliegenden Erfindung ist auch nicht ergänzt mit einer hydrolysierten Proteinquelle, wie Sojabohnen-, Weizen- oder Reispepton oder Hefehydrolysat oder dergleichen.

Unter "Serum" ist im Rahmen dieser Beschreibung ein Blutserum oder ein Immunserum zu verstehen. Dabei versteht man unter Blutserum jenen flüssigen Anteil des Blutes, den man als Überstand erhält, wenn eine Blutprobe zentrifugiert wird. Dieser Überstand enthält bis auf die durch die Gerinnung verbrauchten Gerinnungsfaktoren alle natürlicherweise in der Blutflüssigkeit gelösten Stoffe. Das Blutserum entspricht also dem Blutplasma abzüglich der Gerinnungsfaktoren. Unter Immunserum versteht man eine Aufreinigung spezifischer Antikörper, die aus dem Blutserum immunisierter Säugetiere gewonnen werden.

Seren bedeuten im Rahmen dieser Beschreibung in der Regel Seren von Wirbeltieren, und insbesondere Seren von Kalb, Kuh, Rind, Pferd oder Mensch.

Unter "Zellkulturmedien im Wesentlichen frei von tierischen Bestandteilen" sind im Rahmen der vorliegenden Beschreibung Zellkulturmedien zu verstehen, die keine oder nur geringen Mengen von bis zu 1 Gew. %, vorzugsweise von weniger als 0,1 Gew. %, bezogen auf das Zellkulturmedium an tierischen Bestandteilen enthalten. Typische tierische Bestandteile die in den erfindungsgemäßen Zellkulturen und Zellkulturmedien vermieden werden sind Serum und Serum abgeleitete Proteine, wie z.B. Albumin, Transferrin oder andere Wachstumsfaktoren sowie rekominante Formen davon oder Protein aus Pflanzen- oder Hefehydrolysaten oder ultrafiltrierte Formen davon.

Unter "chemisch definierten Zellkulturmedien" sind im Rahmen der vorliegenden Beschreibung Zellkulturmedien zu verstehen, die neben Wasser zusätzlich chemisch und/oder biotechnologisch hergestellte Bestandteile enthalten, und insbesondere Zellkulturmedien, die neben Wasser ausschließlich aus chemisch und/oder biotechnologisch hergestellten Bestandteilen bestehen. Typischerweise ist ein "chemisch definiertes Zellkulturmedium" (auch "chemisch definiertes Medium" genannt) ein Begriff, der von dem Fachmann auf dem Gebiet der Zellkultur und Zellkulturmedien verstanden wird und dem Fachmann bekannt ist. Demzufolge bezieht sich der Begriff "chemisch definiertes Zellkulturmedium" auf eine Nährlösung, in welcher Zellen enthalten sind und gezüchtet werden und die im Allgemeinen mindestens eine oder mehrere Komponenten aus dem Folgenden bereitstellen: eine Energiequelle (üblicherweise in Form eines Kohlenhydrats wie Glucose); alle essentiellen Aminosäuren und im Allgemeinen die zwanzig basischen Aminosäuren, Säuren, plus Cystein; Vitamine und/oder andere organische Verbindungen, die typischerweise in geringen Konzentrationen benötigt werden; Lipide oder freie Fettsäuren, z.B. Linolsäure; anorganische Verbindungen oder natürlich vorkommende Elemente, die typischerweise in sehr geringen Konzentrationen, üblicherweise im mikromolaren Bereich dem Zellkulturmedium enthalten sind. Zellkulturmedien können auch durch eine Vielzahl von fakultativen Komponenten, wie Salze, z. B. Calcium, Magnesium und Phosphat, und Puffer, z.B. HEPES; Nucleoside und Basen, z.B. Adenosin, Thymidin, Hypoxanthin; Antibiotika, z.B. Gentamycin ergänzt werden.

Der Begriff "chemisch definiertes Zellkulturmedium" steht also für ein vollständig chemisch definiertes Medium, das keine Zusätze aus tierischen Quellen enthält, wie Gewebehydrolysate, z.B. fetales Rinderserum oder dergleichen. Des Weiteren werden Proteine, insbesondere Wachstumsfaktoren wie bspw. Transferrin oder rekombinante Formen davon, ebenfalls nicht zu der Zellkultur gemäß der vorliegenden Erfindung gegeben.

Im Handel erhältliche serum- und/oder protein-freie Zellkulturmedien können in der vorliegenden Erfindung verwendet werden und umfassen zum Beispiel die von der Xell AG (Bielefeld) angebotenen Medien HEK TF (Bestellnr. 861), HEK GM (Bestellnr. 851), HEK FS (Bestellnr. 871), BHK Medium (Bestellnr. 910), BHK FS (Bestelln. 915), MDXK Medium (Bestellnr. 1010), HYB GM (Bestelln. 890), HYB FS (Bestelln. 895), TCX6D Medium (Bestellnr. 1070), TCX10D Medium (Bestellnr. 1100), TCX7D (Bestellnr. 1080), CHO TF (Bestellnr. 886) .

Unter "Bioreaktoren" oder "Fermentern" sind im Rahmen der vorliegenden Beschreibung Gefäße für die Kultivierung von Zellen zu verstehen, in denen die Zellen in Kontakt mit einem Zellkulturmedium stehen und unter dauerhafter Bewegung zu hohen Zelldichten kultiviert werden können. Die Zellen können dabei in dem Zellkulturmedium suspendiert vorliegen oder adhärent auf Oberflächen wachsen, die in Kontakt mit dem Zellkulturmedium stehen. Zweck der Kultivierung in einem Bioreaktor kann die Gewinnung der Zellen oder von Stoffechselprodukten sein. Letztere können z.B. als Wirkstoffe in der pharmazeutischen Industrie oder als Grundchemikalien in der chemischen Industrie eingesetzt werden. In Bioreaktoren werden in der Regel mehrere Faktoren gesteuert und/oder überwacht, die das Wachstum der Zellen beeinflussen. Beispiele dafür sind die Zusammensetzung des Nährmediums, die Sauerstoffzufuhr, die Temperatur, der pH-Wert und die Sterilität. Es können unterschiedliche Reaktorvarianten in unterschiedlicher Ausführung verwendet werden. Beispiele dafür sind Rührkesselreaktoren, z.B. solche aus Metall, die ein Volumen von wenigen bis tausenden Litern haben können und die mit Nährlösung gefüllt werden. Es lassen sich auch ander Varianten einsetzen, wie z.B. Festbettreaktoren, Photobioreaktoren oder Wirbelschichtreaktoren.

Unter "Polymeren" sind im Rahmen der vorliegenden Beschreibung organische Verbindungen zu verstehen, die durch Wiederholung von bestimmten Einheiten (Monomereinheiten oder Wiederholungseinheiten) gekennzeichnet sind. Polymere können aus einer Art oder aus mehreren Arten verschiedener Wiederholungseinheiten bestehen. Polymere werden durch die chemische Reaktion von Monomeren unter Ausbildung von kovalenten Bindungen hergestellt (Polymerisation) und bilden durch Verknüpfen der polymerisierten Einheiten das sogenannte Polymerrückgrad. Dieses kann Seitenketten aufweisen, an denen sich funktionelle Gruppen befinden können. Homopolymere bestehen nur aus einer Monomereinheit. Copolymere bestehen hingegen aus mindestens zwei unterschiedlichen Monomereinheiten, welche statistisch, als Gradient, alternierend oder als Block angeordnet sein können.

Unter "Tensiden" sind im Rahmen der vorliegenden Beschreibung wasserlösliche Stoffe oder Stoffgemische zu verstehen, die der Stabilisierung von Zellkulturen dienen. Sie werden üblicherweise der wässrigen Phase bei der Kultivierung der Zellen zugegeben und dienen vor allem dazu, die Auswirkungen von Scherkräften auf die Zellen zu minimieren und damit die Viabilität der Zellen zu erhöhen.

Unter "wasserlöslichen Poly(oxazolin)en" sind im Rahmen der vorliegenden Beschreibung Polyoxazoline zu verstehen, die sich zu mindestens 10 g/L Wasser bei 25 °C lösen.

Die erfindungsgemäß eingesetzten Zellen können nach Standardmethoden erzeugt und kultiviert werden.

So lassen sich z.B. Primärkulturen aus unterschiedlichen Geweben anlegen, beispielsweise aus Geweben einzelner Organe, wie Haut, Herz, Niere oder Leber, oder aus Tumorgewebe. Die Gewebezellen können durch an sich bekannte Methoden vereinzelt werden, z.B. durch Behandlung wird mit einer Protease, wodurch die Proteine abgebaut werden, die den Zellverband aufrechterhalten. Es kann auch angebracht sein, durch Zugabe von Wachstumsfaktoren gezielt manche Zelltypen zur Teilung anzueregen oder im Fall von schlecht wachsenden Zelltypen Fütterzellen, basalmembranartige Matrices oder recombinate Bestandteile der extrazellulären Matrix zu verwenden. Die erfindungsgemäß eingesetzten Zellen können auch durch Einschleusung eines Plasmids als Vektor genetisch verändert werden.

Die erfindungsgemäß eingesetzten Zellen können eine eingeschränkte Lebensdauer besitzen oder es handelt sich um unsterbliche Zelllinien mit der Fähigkeit, sich unendlich zu teilen. Diese können durch durch zufällige Mutation erzeugt worden sein, z.B. in Tumorzellen, oder durch gezielte Veränderung, beispielsweise durch die künstliche Expression des Telomerase-Gens.

Bei der erfindungsgemäß eingesetzten Zellen kann es sich um adhärent (auf Oberflächen) wachsende Zellen handeln, wie beispielsweise Fibroblasten, Endothelzellen oder Knorpelzellen, oder es kann sich um Supensionszellen handeln, die frei im Nährmedium schwimmend wachsen, wie zum Beispiel Lymphozyten.

Bevorzugt werden Zellen eingesetzt, die im Zellkulturmedium suspendiert vorliegen.

Besonders bevorzugt werden suspensions-adaptierte Zellen eingesetzt. Dabei handelt es sich um Zellen, vorzugsweise um eukaryontische Zellen, die originär adhärent wachsen und kultiviert werden, aber durch Änderung der Medienbestandteile und Kultivierung in Suspension gehen können. Dadurch lassen sich höhere Zellkulturdichten erreichen.

Kulturbedingungen und Zellkulturmedien werden in Abhängigkeit von den einzelnen kultivierten Zellen ausgewählt. Die verschiedenen Zelltypen bevorzugen dabei unterschiedliche Nährmedien, die spezifisch zusammengestellt werden. So werden beispielsweise unterschiedliche pH-Werte eingestellt und die einzelnen Nährmedien können unterschiedliche Aminosäuren und/oder andere Nährstoffe in unterschiedlichen Konzentrationen enthalten.

Die erfindungsgemäßen Zellkulturen werden besonders auf dem Gebiet der Biotechnologie eingesetzt. Dabei kann es sich um die Produktion von (rekombinanten) Proteinen, Viren- und/oder Viruspartikelproduktion, Untersuchung von Stoffwechsel, Teilung und weiteren zellulären Prozessen handeln. Weiterhin können die erfindungsgemäßen Zellkulturen als Testsysteme eingesetzt werden, beispielsweise bei der Untersuchung der Wirkung von Substanzen auf Zelleigenschaften, wie die Signaltransduktion oder die Toxizität. Die erfindungsgemäßen Zellkulturen können auch für die Herstellung von biotechnischen Produkten verwendet werden. Beispielsweise zur Herstellung von chemischen Verbindungen, wie von Grundstoffchemikalien oder von Wirkstoffen für die Pharmazeutik, beispielsweise von monoklonalen Antikörpern, Proteinen oder Impfstoffen. Die erfindungsgemäßen Zellkulturen können auch in der Pflanzenzucht eingesetzt werden, beispielsweise in der pflanzlichen Gewebekultur, bei der aus Zellkulturen komplette Pflanzen erzeugt werden können.

Bevorzugt werden in den erfindungsgemäßen Zellkulturen die folgenden Zelllinien eingesetzt:
293-T: Ursprungsspezies Mensch, Ursprungsgewebe Niere, Morphologie Epithel A431: Ursprungsspezies Mensch, Ursprungsgewebe Haut, Morphologie Epithel A549: Ursprungsspezies Mensch, Ursprungsgewebe Adenokarzinomo der Lunge, Morphologie Epithel
BCP1: Ursprungsspezies Mensch, Ursprungsgewebe Blut, Morphologie Lymphozyt bEnd.3: Ursprungsspezies Maus, Ursprungsgewebe Gehirn/Großhirnrinde, Morphologie Endothel
BHK-21: Ursprungsspezies Hamster, Ursprungsgewebe Niere (embryonal), Morphologie Fibroblast
BxPC-3: Ursprungsspezies Mensch; Ursprungsgewebe Pankreas Adenokarzinom, Morphologie Epithel
BY-2: Ursprungsspezies Tabak, Ursprungsgewebe Am Keimling induzierter Kallus CHO: Ursprungsspezies Hamster, Ursprungsgewebe Ovarien, Morphologie Epithel CMT: Ursprungsspezies Hund, Ursprungsgewebe Brsutdüse, Morphologie Epithel COS-1: Ursprungsspezies Affe, Ursprungsgewebe Niere, Morphologie Fibroblast COS-7: Ursprungsspezies Affe, Ursprungsgewebe Niere, Morphologie Fibroblast CV-1: Ursprungsspezies Affe, Ursprungsgewebe Niere, Morphologie Fibroblast EPC: Ursprungsspezies Fisch, Ursprungsgewebe Haut, Morphologie Epithel HDMEC-T: Ursprungsspezies Mensch, Ursprungsgewebe Vorhaut, Morphologie Endothel
HEK oder HEK 293: Ursprungsspezies Mensch, Ursprungsgewebe Niere (embryonal), Morphologie Epithel
HeLa: Ursprungsspezies Mensch, Ursprungsgewebe Zervixkarzinom, Morphologie Epithel
HepG2: Ursprungsspezies Mensch, Ursprungsgewebe Leberzellkarzinom, Morphologie Epithel
HL-60: Ursprungsspezies Mensch, Ursprungsgewebe Promyeloblasten, Morphologie Blutzellen
HMEC-1: Ursprungsspezies Mensch, Ursprungsgewebe Vorhaut, Morphologie Endothel
HUVEC-T: Ursprungsspezies Mensch, Ursprungsgewebe Nabelschnurvene, Morphologie Endothel
HT-1080: Ursprungsspezies Mensch, Ursprungsgewebe Fibrosarkom, Morphologie Bindegewebszellen
Jurkat: Ursprungsspezies Mensch, Ursprungsgewebe T-Zell-Leukämie, Morphologie Blutzellen
K562: Ursprungsspezies Mensch, Ursprungsgewebe Leukämie, Morphologie myeloische Blutzellen
LNCaP: Ursprungsspezies Mensch, Ursprungsgewebe Prostata, Morphologie Epithel
MCF-7: Ursprungsspezies Mensch, Ursprungsgewebe Brust Adenokarzinom, Morphologie Epithel
MCF-10A: Ursprungsspezies Mensch, Ursprungsgewebe Brustdrüse, Morphologie Epithel
MDCK II: Ursprungsspezies Hund, Ursprungsgewebe Niere, Morphologie Epithel MDT-1A: Ursprungsspezies Maus, Morphologie Epithel
MyEnd: Ursprungsspezies Maus, Morphologie Endothel
Neuro-2A: Ursprungsspezies Maus, Ursprungsgewebe Gehirn, Morphologie Neuroblast
NIH-3T3-T: Ursprungsspezies Maus, Ursprungsgewebe Embryo, Morphologie Fibroblast
NTERA-2 cl.D1: Ursprungsspezies Mensch, Ursprungsgewebe Hoden Lungenmetastase, Morphologie Epithel
P19: Ursprungsspezies Maus, Ursprungsgewebe embryonales Karzinom, Morphologie Epithel
PANC-1: Ursprungsspezies Mensch, Ursprungsgewebe Pankreas Adenokarzinom, Morphologie Epithel
Peer: Ursprungsspezies Mensch, Ursprungsgewebe T-cell leukemia
RTL-W1-T: Ursprungsspezies Regenbogenforelle, Morphologie Fibroblast
Sf-9: Ursprungsspezies Nachtfalter, Ursprungsgewebe Ovar
Saos-2: Ursprungsspezies Mensch, Ursprungsgewebe Osteosarkom, Morphologie Epithel
T2: Ursprungsspezies Mensch, Morphologie T-cell leukemia
T84: Ursprungsspezies Mensch, Ursprungsgewebe Kolorektales Karzinom Lungenmetastase, Morphologie Epithel
U-937: Ursprungsspezies Mensch, Ursprungsgewebe Burkitt-Lymphom, Morphologie 'monozytär.

Weitere in den erfindungsgemäßen Zellkulturen bevorzugt eingesetzte Zellen sind solche, die keine Fibroblasten, Pankreaszellen oder Makrophagen sind. In den erfindungsgemäßen Zellkulturen besonders bevorzugt eingesetze Zellen sind solche, die keine 3T3 Fibroblasten, die βT3 Pankreaszellen und die murinen P388.D1 Monozyten/Makrophagen sind.

Weitere in den erfindungsgemäßen Zellkulturen bevorzugt eingesetzte Zellen sind Hybridomazellen. Dabei handelt es sich bekanntermaßen um Wirkstoffproduzierende Zellen, die mit Krebszellen (immortalisierten Zellen) fusioniert worden sind, wodurch unsterbliche Hybride entstehen.

Weitere in den erfindungsgemäßen Zellkulturen bevorzugt eingesetzte Zellen sind Stammzellen. Dabei handelt es sich bekanntermaßen um Körperzellen, die sich in verschiedene Zelltypen oder Gewebe ausdifferenzieren können.

Besonders bevorzugt ist der Einsatz der Zelllinien CHO und HEK, vorzugsweise HEK 293.

Die erfindungsgemäßen Zellkulturen enthalten ein oder mehrere wasserlösliche Poly(oxazolin)e. Die Menge an Poly(oxazolin)en, bezogen auf die Gesamtmenge der erfindungsgemäßen Zellkultur, beträgt in der Regel 0,01 bis 15 Gew. %, 0,1 bis 15 Gew.%, vorzugsweise 0,05 bis 10 Gew. %, mehr bevorzugt 0,075, 0,08, 0,085, 0,09, 0,095, 0,1, 0,15, 0,2, 0,25, 0,3, 0,35, 0,4, 0,45, 0,5, 0,55, 0,6, 0,65, 0,7, 0,75, 0,8, 0,85, 0,9, 0,95, 1, 1,5, 2, 2,5, 3, 3,5, 4, 4,5, 5, 5,5, 6,6,5, 7, 7,5, 8, 8,5, 9, 9,5, 10, 11, 12, 13, 14 bis 15 Gew.%, ganz besonders bevorzugt 0,07, 0,075, 0,08, 0,085, 0,09, 0,095, 0,1, 0,15, 0,2, 0,25, 0,3, 0,35, 0,4, 0,45, 0,5, 0,55, 0,6, 0,65, 0,7, 0,75, 0,8, 0,85, 0,9, 0,95, 1, 2, 3, 4, 5, 6, 7, 8, 9 bis 10 Gew. % und äußerst bevorzugt 0,1, 0,15, 0,2, 0,25, 0,3, 0,35, 0,4, 0,45, 0,5, 0,55, 0,6, 0,65, 0,7, 0,75, 0,8, 0,85, 0,9, 0,95, 1, 2, 3, 4, 5, 6, 7, 8, 9 bis 10 Gew. %.

Poly(oxazolin)e sind bekannte Verbindungen. Diese werden üblicherweise durch kationische Ringöffnungspolymerisation von Oxazolinen, vorzugsweise von 2-Oxazolinen, in Lösung und in Gegenwart eines Initiators hergestellt. Beispiele für Initiatoren sind Elektrophile, wie Salze oder Ester von aromatischen Sulfonsäuren oder Carbonsäuren oder Salze oder Ester von aliphatischen Sulfonsäuren oder Carbonsäuren oder aromatische Halogenverbindungen. Es können auch mehrfachfunktionelle Elektrophile als Initiatoren eingesetzt werden. Dabei können neben linearen Poly(oxazolin)en auch verzweigte oder sternförmige Moleküle entstehen. Beispiele für bevorzugte Initiatoren sind Ester der Arylsulfonsäuren, wie Methyltosylat, Ester der Alkansulfonsäuren, wie Trifluormethansulfonsäure, oder Mono- oder Dibrombenzol. Die Polymerisation wird üblicherweise in einem polaren aprotischen Lösungsmittel durchgeführt, beispielsweise in Acetonitril.

Als Oxazoline zur Herstellung der erfindungsgemäß eingesetzten Poly(oxazolin)e werden 2-Oxazoline (4,5-Dihydrooxazole) mit einer C=N-Doppelbindung zwischen dem Kohlenstoffatom 2 und dem Stickstoffatom eingesetzt. Diese können am 2-, 4- und/oder 5-Kohlenstoffatom und/oder am 3-Stickstoffatom substituiert sein, vorzugsweise am 2-Kohlenstoffatom und/oder am 3-Stickstoffatom.

Bevorzugt werden 2-Oxazoline eingesetzt, welche an 2-Position einen Substituenten enthalten. Beispiele für solche Substituenten sind Methyl oder Ethyl.

Neben den 2-Oxazolinen können bei der Herstellung der erfindungsgemäß eingesetzten wasserlöslichen Poly(oxazolin)e noch geringe Mengen weiterer mit 2-Oxazolinen copolymerisierbarer Monomere eingesetzt werden.

Die erfindungsgemäß eingesetzten wasserlöslichen Poly(oxazolin)e enthalten in der Regel mindestens 80 Gew. %, insbesondere mindestens 90 Gew. % und ganz besonders bevorzugt mindestens 95 Gew. %, bezogen auf deren Gesamtmasse, an wiederkehrenden Struktureinheiten der Formel I und/oder der Formel II

-NR¹-CR³H-CR⁴H- (I),

-NR¹-CR³H-CR⁴H-CR⁵H- (II),

worin
R¹ einen Rest der Formel -CO-R² bedeutet,
R³, R⁴ und R⁵ unabhängig voneinander Wasserstoff, Methyl, Ethyl, Propyl oder Butyl bedeuten,
R² ausgewählt wird aus der Gruppe bestehend aus Wasserstoff, Methyl, Ethyl, -CₘH₂ₘ-X oder -(CₙH₂ₙ-O)ₒ-(CₚH₂ₚ-O)_{q}-R⁶,
R⁶ Wasserstoff oder C₁-C₆-Alkyl, insbesondere Methyl oder ganz besonders bevorzugt Wasserstoff ist,
m eine ganze Zahl von 1 bis 6 ist,
X ausgewählt wird aus der Gruppe bestehend aus Hydroxyl, Alkoxy, Amino, N-Alkylamino, N, N-Dialkylamino, Carboxyl, Carbonsäureester, Sulfonyl, Sulfonsäureester oder Carbamat,
n und p unabhängig voneinander ganze Zahlen von 2 bis 4 sind, wobei n ungleich p ist, n vorzugsweise 2 ist und p vorzugsweise 3 bedeutet, und
o und q unabhängig ganze Zahlen von 0 bis 60, insbesondere 1 bis 20 und ganz besonders bevorzugt 2 bis 10 sind, wobei mindestens eines der o oder q ungleich 0 ist.

Bevorzugte erfindungsgemäß eingesetzte wasserlösliche Poly(oxazolin)e sind solche, in denen R² Wasserstoff, Methyl oder Ethyl ist und R³ bis R⁵ Wasserstoff bedeuten oder in denen R² Wasserstoff, Methyl oder Ethyl ist und zwei der Reste R³ bis R⁵ Wasserstoff sind ist und einer der Reste R³ bis R⁵ Methyl oder Ethyl ist.

Die Molmasse der erfindungsgemäß eingesetzten Poly(oxazolin)e beträgt in der Regel 2.500 bis 500.000 g/mol, insbesondere 5.000 bis 50.000 g/mol. Die Molmasse wird für die Zwecke der vorliegenden Beschreibung durch ¹H-NMR-Analyse bestimmt. Eine indirekte Bestimmung der Molmasse ist auch über Endgruppenbestimmung des Polymerisationsgrades und des Molekulargewichts des Monomers möglich, indem man die Integrale der Protonen minteinander vergleicht und so die Anzahl der Wiederholeinheiten bestimmt.

Ganz besonders bevorzugte erfindungsgemäße Zellkulturen enthalten ein wasserlösliches Poly(oxazolin), das mindestens 90 Gew. %, insbesondere mindestens 95 Gew. %, bezogen auf dessen Gesamtmasse, an wiederkehrenden Struktureinheiten der Formel I aufweist, worin R² Methyl oder Ethyl bedeutet.

Weitere bevorzugte erfindungsgemäße Zellkulturen enthalten neben den Zellen, dem Zellkulturmedium, dem wasserlöslichen Poly(oxazolin) einen oder mehrere Wirkstoffe, insbesondere einen oder mehrere pharmazeutische Wirkstoffe.

Die Kultivierung der erfindungsgemäßen Zellkulturen kann in an sich bekannter Weise erfolgen. Dabei werden Bioreaktoren eingesetzt. Diese werden mit Nährmedium und Zellen gefüllt. Die Kultivierung kann ansatzweise oder kontinuierlich erfolgen. Der Inhalt des Bioreaktors wird turbulent bewegt, vorzugsweise durch Rühren, wobei zur Erzeugung von Turbulenzen vorteilhaft Stromstörer eingesetzt werden. Das Bewegen des Reaktorinhalts kann durch an sich bekannte Rührvorrichtungen erfolgen und/oder durch Eindüsen von Flüssigkeiten.

Die Erfindung betrifft auch Verfahren zur Proteinproduktion, Viren- und/oder Viruspartikelproduktion, Untersuchung von Stoffwechsel, Teilung und/oder weiteren zellulären Prozessen, umfassend die Kultivierung der hierin beschriebenen erfindungsgemäßen Zellkulturen.

Die hierin beschriebenen Verfahren enthalten ferner bevorzugt die folgenden Maßnahmen:
i) Befüllen eines Bioreaktors mit Zellen und einem hierin beschriebenen Zellkulturmedium, vorzugsweise einem serum- und/oder protein-freien Zellkulturmedium, wobei das Zellkulturmedium ein oder mehrere hierin beschriebene wasserlösliche Poly(oxazolin)e enthält; und
ii) turbulentes Bewegen des Inhalts des Bioreaktors.

Die Temperatur bei der Kultivierung sowie die Zusammensetzung des Nährmediums werden nach den Bedürfnissen der zu kultivierenden Zellen ausgewählt.

Vorzugsweise erfolgt die Kultivierung der Zellen bei Temperaturen zwischen 31 und 39 °C, insbesondere bei Temperaturen zwischen 36 und 37 °C. Das Kultivieren der erfindungsgemäßen Zellkulturen kann anaerob oder insbesondere aerob erfolgen. Typischerweise enthält die Atmosphäre neben Stickstoff, Sauerstoff und Edelgasen noch CO₂, beispielsweise in Mengen von 0,01 bis 10 Gew. %, insbesondere in Mengen von 0,01 bis 5 Gew. %, bezogen auf die Masse der Atmosphäre. Bevorzugt besteht die Atmosphäre im Bioreaktor aus Luft.

Die Dauer der Kultivierung der Zellen kann in weitem Bereich gewählt werden und sowohl satzweise wie auch kontinuierlich mit und ohne Zufütterung von Nährstoffen erfolgen. Typische Kultivierungsdauern für satzweise Verfahren betragen 5 Stunden bis 30 Tage, vorzugsweise 5 Stunden bis 21 Tage und insbesondere 10 Stunden bis 15 Tage. Typische Kultivierungsdauern für kontinuierliche Verfahren betragen 10 Tage bis 180 Tage, vorzugsweise 10 Tage bis 60 Tage und insbesondere 15 Tage bis 35 Tage.

Je nach Teilungsrate und Dichte der Zellen können Zellverbände alle paar Tage gelöst und auf neue Gefäße verteilt werden (auch "Passage" genannt). Die Passagenzahl gibt dabei die Häufigkeit an, mit der die Zellen bereits passagiert wurden. Bei adhärenten Zellen in kontinuierlicher Kultur werden die Zellen vorzugsweise regelmäßig vereinzelt, um eine Konfluenz und die damit verbundene Zellkontakthemmung zu vermeiden.

Die erfindungsgemäß eingesetzten Poly(oxazolin)e können dem Zellkulturmedium während der Kultivierung und/oder während einer Modifikation der Zellkultur, wie einer Transfektion, zugesetzt werden.

Nach der Kultivierung der erfindungsgemäßen Zellkultur im Bioreaktor wird diese vorzugsweise aufgearbeitet. Dabei werden die Zellen und/oder die erzeugten Wirkstoffe von den übrigen Inhaltsstoffen abgetrennt. Dieses kann durch Standardmethoden erfolgen, beispielsweise durch Filtration oder Zentrifugieren. Die Erfindung betrifft auch die Verwendung der hierin beschriebenen wasserlöslichem Poly(oxazolin)e zur Stabilisierung von turbulent bewegten Zellkulturen. Bevorzugt werden, wie hierin vorstehend beschrieben, die Poly(oxazolin)e in ein hierin beschriebenes Zellkulturmedium eingebracht.

Die folgenden Beispiele erläutern die Erfindung ohne diese zu begrenzen.

### Beispiel 1A: Herstellung von Poly(2-oxazolin)en (POₓ) in der Mikrowelle

Die Synthese von Poly(2-oxazolin)en wurde bereits in der Literatur beschrieben (vergl. z.B. F. Wiesbrock et al. Macromolecular Rapid Communications 2004, 25, 1895-1899). Die Vorgehensweise wird deshalb beispielhaft für Poly(2-ethyl-2-oxazolin) mit einem Polymerisationsgrad (DP) von 61 (P(EtOₓ)₆₁) beschrieben.

In einem Mikrowellenreaktionsgefäß wurden unter inerten Bedingungen 2-Ethyl- -2-oxazolin (6,06 mL, 60,0 mmol), Methyltosylat (0,15 mL, 0,1 mmol) und Acetonitril (8,79 mL) gemischt. Das Reaktionsgefäß wurde dann in einer Synthesemikrowelle für 14 min auf 140 °C erhitzt. Anschließend wurde die Reaktion mittels der Zugabe von 0,5 mL deionisiertem Wasser terminiert und über Nacht bei Raumtemperatur gerührt. Die resultierende Lösung wurde aufgereinigt, indem sie mit Dichlormethan verdünnt und dann in einem Überschuss eiskaltem Diethylether gefällt wurde. Das ausgefallene Polymer wurde dann abfiltriert und in Dichlormethan gelöst. Das Lösungsmittel wurde anschließend am Rotationsverdampfer entfernt und das Polymer bis zur vollständigen Lösungsmittelfreiheit am Hochvakuum getrocknet. Das finale Produkt lag als kristalliner, weißer Feststoff vor.

¹H-NMR (CDCl₃, 300 MHz): δ = 4,34 (0,1H, s, Rückgrat-OH), 3,44 (4,0H, s, Rückgrat), 3,02 (0,3H, s, CH₃-Rückgrat), 2,4 (1,7H, m, CH₂ (EtOx)), 1,11 (2,5H, s, CH₃ (EtOx)) ppm.

SEC (Eluent: DMAc¹), 0,21% LiCl, PS²)-Standard): Mₙ = 11.200 g mol⁻¹, M_{w} = 12.200 g mol⁻¹, Ð = 1,09.
¹⁾ DMAc = Dimethylacetamid
²⁾ PS = Polystyrol

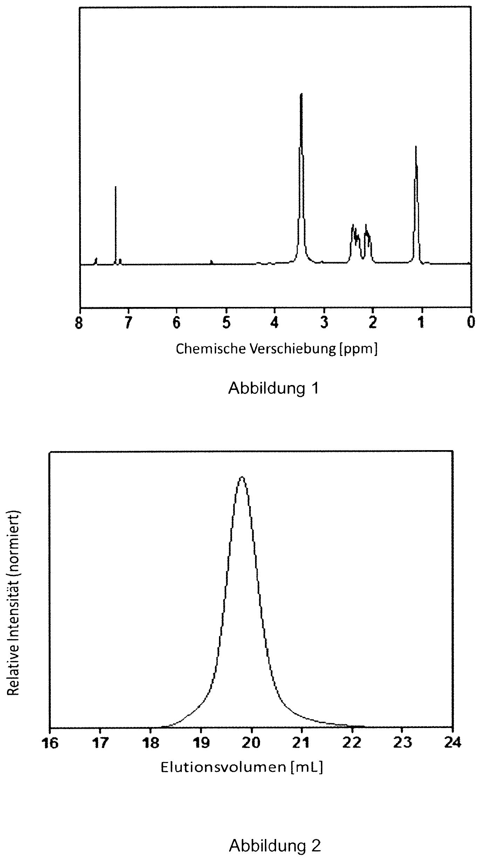

Abbildung 1 zeigt ein ¹H-NMR (300 MHz, CDCl₃) des aufgereinigten P(EtOx)₆₁.

Abbildung 2 zeigt ein SEC Elugramm (DMAc, 0,21% LiCl, PS-Kalibration) des aufgereinigten P (EtOx)₆₁.

### Beispiel 1B: Herstellung von POₓ im Reaktor

Im Reaktor wurden unter inerten Bedingungen 2-Ethyl-2-oxazolin (4,04 L, 40,0 mol), Methyltosylat (100 mL, 0,67 mol) und Acetonitril (5,86 L) gemischt. Der Reaktor wurde dann unter Rückfluß für 6 Std erhitzt. Der Reaktionsfortschritt wurde kontrolliert, indem in regelmäßigen Abständen Proben gezogen wurden. Anschließend wurde die Reaktion mittels der Zugabe von 270 mL deionisiertem Wasser terminiert und über Nacht bei Raumtemperatur gerührt. Die resultierende Lösung wurde in fünf Portionen aufgereinigt. Dazu wurde jeweils das Lösungsmittel am Rotationsverdampfer entfernt. Anschließend wurde das Polymer in 4 L Dichlormethan gelöst. Dann wurde die organische Phase mit 2 L einer gesättigten Natriumhydrogencarbonat Lösung und anschließend zweimal mit 2 L einer gesättigten Natriumchlorid Lösung gewaschen. Die organische Phase wurde über Natriumsulfat getrocknet und das Lösungsmittel am Rotationsverdampfer verdampft. Dann wurde das Polymer bis zur vollständigen Lösungsmittelfreiheit am Hochvakuum getrocknet. Das finale Produkt lag als kristalliner, weißer Feststoff vor.

### Beispiel 2: Zellwachstum in Schikanekolben

Suspensions-adaptierte HEK-F Zellen wurden mittels dynamischer Zellkultur in serum freiem Medium (Mangelvariante des HEK TF Mediums (serum und protein frei, ohne Pluronic^{®} F68) (Bestellnr. 861 (Xell AG, Bielefeld)) für mindestens 5 Tage ohne Zugabe von neuem Medium kultiviert. In einem Schikanekolben (250 mL) wurden die Zellen mit einer Zelldichte von 0,3 × 10⁶ Zellen pro mL in 30 - 40 mL Minimalmedium mit entsprechend zugesetztem Tensid (750 mg L⁻¹) angesetzt. Die Kultivierung erfolgte im Inkubator unter Schütteln bei 128 rpm, 37°C und 5% CO₂. Jeden Tag wurde die Zelldichte sowie Zellviabilität durch Auszählung mittels Trypanblau ermittelt. Als Abbruchkriterium gilt eine Zellviabilität unter 60%.

**Tabelle 1: Zellkonzentrationen der lebenden Zellen angegeben in Zellen pro Milliliter**

| Tag | Pluronic^{®} F68 | P(EtOx)₆₁ | P(MeOx)₅₇ |
|---|---|---|---|
| 0 | 300000 | 300000 | 300000 |
| 1 | 980000 | 720000 | 1400000 |
| 2 | 1644000 | 1816000 | 2012000 |
| 3 | 3045000 | 3308000 | 3044000 |
| 4 | 3728000 | 4304000 | 4904000 |
| 5 | 5296000 | 6152000 | 6040000 |
| 6 | 6030000 | 6960000 | 6488000 |
| 7 | 5670000 | 7288000 | 6752000 |
| 8 | 8020000 | 7640000 | 7820000 |

**Tabelle 2: Überlebensrate der Zellen, angegeben in %**

| Tag | Pluronic^{®} F68 | P(EtOx)₆₁^{a)} | P(MeOx)₅₇^{b)} |
|---|---|---|---|
| 0 | 100 | 100 | 100 |
| 1 | 96,1 | 97,3 | 93,3 |
| 2 | 97,2 | 96,8 | 97,7 |
| 3 | 95,4 | 98,2 | 98,0 |
| 4 | 96,5 | 98,3 | 95,8 |
| 5 | 96,2 | 95,1 | 94,0 |
| 6 | 93,6 | 95,1 | 97,7 |
| 7 | 89,4 | 95,6 | 96,8 |
| 8 | 92,7 | 90,4 | 93,3 |

| | | | |
|---|---|---|---|
| ^{a)} Poly(2-ethyl-2-oxazolin) mit einem Polymerisationsgrad (DP) von 61 ^{b)} Poly(2-methyl-2-oxazolin) mit einem Polymerisationsgrad (DP) von 57 | | | |

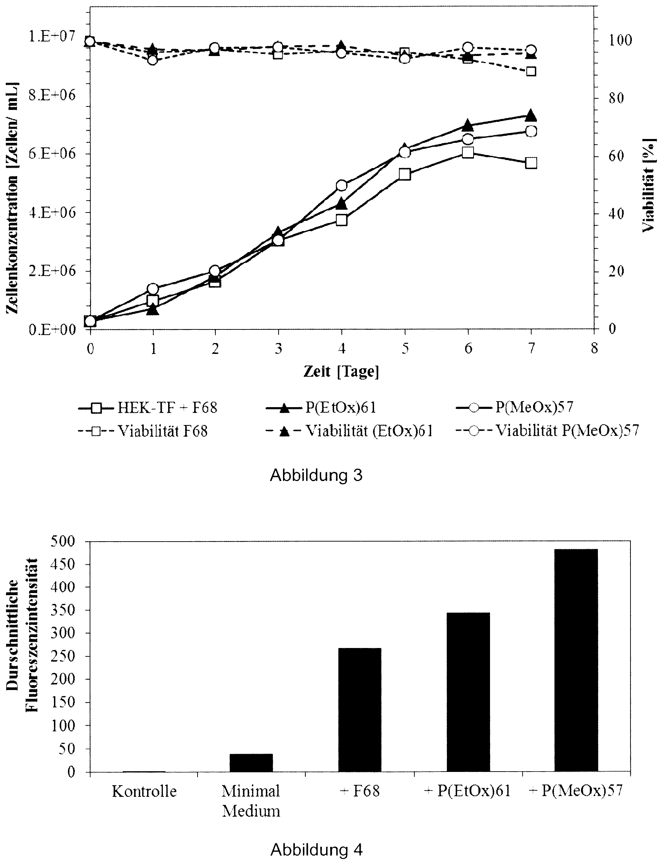

Abbildung 3 zeigt die Zellkonzentrationen und Viabilität der HEK-F Zellen bei Kultivierung mit unterschiedlichen Tensiden.

### Beispiel 3: Transfektion von Suspensionszellen

HEK-F Zellen wurden im entsprechenden Minimalmedium Mangelvariante des HEK TF Mediums (serum und protein frei, ohne Pluronic^{®} F68) (Bestellnr. 861 (Xell AG, Bielefeld)) mit Tensid (750 mg L⁻¹) vorkultiviert. Am Tag der Transfektion wurden die Zellen zentrifugiert und die Zelldichte mit frischem Medium auf 3 × 10⁶ Zellen mL⁻¹ eingestellt. Die Transfektion bei N/P 20 (Amin- zu Phosphatverhältnis) erfolgte in einem 2 mL Ansatz wie folgt: zunächst Zugabe von 15 µg mL⁻¹ pDNA (EGFP Reportergen) zur Zellsuspension mit anschließendem Schwenken dieser, danach Zugabe von Polyethylenimin (PEI, 1 mg mL⁻¹) und wiederholtem Schwenken der Kultur. Der Ansatz wurden für 4 h unter Schütteln im Inkubator bei 37°C, 5% CO₂ inkubiert. Nach dieser Inkubationsperiode wurden die Zellen in 6-Wellplatten überführt und mit gleichen Volumen frischen Mediums verdünnt sowie für weitere 48 h inkubiert. Die Bestimmung der Transfektionseffizienz erfolgte mittels Durchflusszytometrie.

**Tabelle 3 zeigt die Ergebnisse der Transfektionsexperimente an HEK-F Zellen in Minimalmedium**

| | Inkubationszeit [Stunden] | Zellviabilität [%] | Transfizierte Zellen [%] | Durchschnittliche Fluoreszenzintensität |
|---|---|---|---|---|
| Negativkontrolle | 48 | 98,4 | 0,7 | 0,775 |
| Minimalmedium | 48 | 95,7 | 11,7 | 37,700 |
| Pluoronic^{®} F68 | 48 | 96,6 | 49,0 | 267,000 |
| P(EtOx)₆₁ | 48 | 96,3 | 60,3 | 344,000 |
| P(MeOx)₅₇ | 48 | 97,7 | 74,8 | 482,000 |

Abbildung 4 zeigt die durchschnittliche Fluoreszenzintensität der transfizierten HEK-F Zellen.

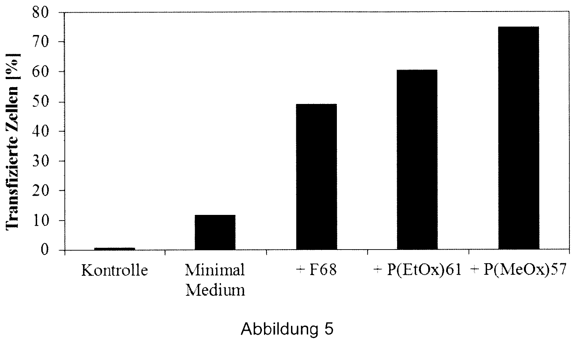

Abbildung 5 zeigt eine Übersicht der Anzahl der transifizierten Zellen, angegeben in %, bei der Verwendung unterschiedlicher Kulturmedien.

### Beispiel 4: Variation des Konzentrationsbereichs von PO_{X}

Um den Konzentrationsbereich einschätzen zu können, in dem PO_{X} als Surfactant in Zellkulturprozessen verwendet werden kann, wurden zusätzliche Kultivierungen durchgeführt. HEK F Zellen wurden hierzu in einer Mangelvariante des HEK TF Mediums (serum und protein frei, ohne Pluronic^{®} F68) (Bestellnr. 861 (Xell AG, Bielefeld)) unter Zusatz des derzeitigen Standard-Surfactants Pluronic^{®} F68 oder des P(EtOx) kultiviert. Dabei wurden für Pluronic^{®} F68 Konzentrationen von 0,75 g/L und 1 g/L eingesetzt (am häufigsten verwendete Konzentrationen) und P(EtOx) wurde in einem größeren Konzentrationsbereich von 0,75 g/L bis 15 g/L getestet. Um darüber hinaus auch die protektive Eigenschaft gegenüber erhöhten Scherkräften zu untersuchen, wurde diese Testung sowohl in Schüttelkolben ohne Schikane als auch mit Schikane durchgeführt. Die Ergebnisse sind in den untenstehenden Graphen in den Abbildungen 6 bis 8 dargestellt.

In den Abbildungen 6 bis 13 wird P(EtOx) mit A60 bezeichnet.

Abbildung 6 gibt einen Überblick der Wachstumskurven und des Viabilitätsverlaufs (gestrichelte Linien) der HEK F Zellen in HEK TF Medium. Als Surfactant wurden verschiedenen Konzentrationen Pluronic^{®} F68 (0,75 g/L und 1 g/L) und P(EtOx) (0,75-15 g/L) eingesetzt. Je Surfactant und Konzentration wurden ein Schüttelkolben mit Schikane (baffled, gepunktete Linien) und ein Schüttelkolben ohne Schikane (plain, durchgezogene Linien) eingesetzt, um die Zellen verschiedenen Scherkräften auszusetzen

Abbildung 6 zeigt eine Gesamtübersicht der Konzentrationstestung von Pluronic^{®} F68 gegen P(EtOx). Generell waren die maximalen Zelldichten in den Schüttelkolben ohne Schikane deutlich erhöht gegenüber denen in Schüttelkolben mit Schikane (9,6-12,9·10⁶ Zellen/mL gegenüber 0,2-5,6·10⁶ Zellen/mL). Auffällig ist hierbei, dass die Zellen in Kultivierung mit P(EtOx) durchweg höhere Zelldichten erreichten als in Kultivierungen mit Pluronic^{®} F68. Besonders unter hohen Scherbelastungen zeigten die Kulturen mit dem Surfactant PO_{X} deutlich erhöhte maximale Zelldichten, erhöhte Viabilitäten und eine längere Kulturdauer gegenüber dem bislang verwendeten Pluronic^{®} F68. Die Viabilität der Kulturen mit Pluronic F68 sank innerhalb von 3 bzw. 4 Tagen auf unter 60 %.

Hinsichtlich des Konzentrationsbereiches, in dem P(EtOx) hier getestet wurde, zeigten sich in den Schüttelkolben ohne Schikane kaum Unterschiede in der maximalen Lebendzelldichte (10,8-12,6·10⁶ Zellen/mL). Die Kulturen mit erhöhtem Scherstress zeigten maximale Lebendzelldichten von 3,5-5,6·10⁶ Zellen/mL, wobei kein direkter Bezug zur jeweils eingesetzten Konzentration gesehen werden konnte.

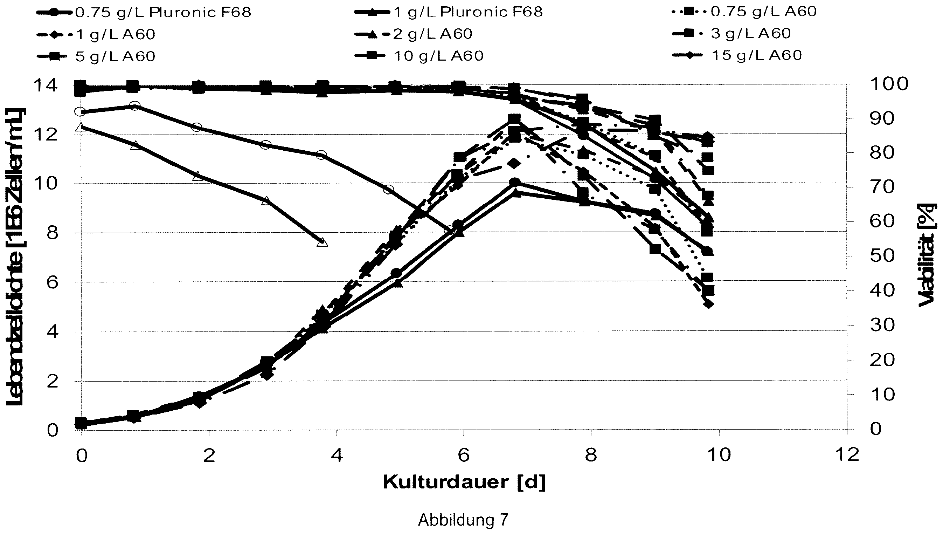

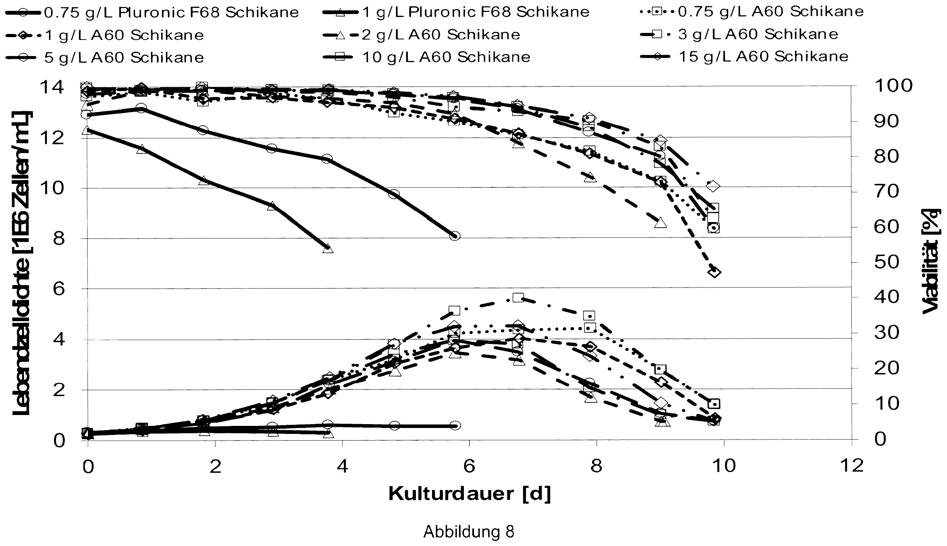

Für einen besseren Einblick sind die Kulturen in den Abbildungen 7 und 8 nochmals nach Scherbelastung getrennt aufgeführt.

Abbildung 7 zeigt Wachstumskurven (durchgezogene Linien) und Viabilitätsverlauf (gestrichelte Linien) der HEK F Zellen in HEK TF Medium in Schüttelkolben ohne Schikane. Als Surfactant wurden verschiedenen Konzentrationen Pluronic^{®} F68 (0,75 g/L und 1 g/L) und P(EtOx) (0,75-15 g/L) eingesetzt.

Abbildung 8 zeigt Wachstumskurven (durchgezogene Linien) und Viabilitätsverlauf (gestrichelte Linien) der HEK F Zellen in HEK TF Medium in Schüttelkolben mit Schikane (baffled). Als Surfactant wurden verschiedenen Konzentrationen Pluronic^{®} F68 (0,75 g/L und 1 g/L) und P(EtOx) (0,75-15 g/L) eingesetzt.

### Beispiel 5: Lot-Testung PO_{X}

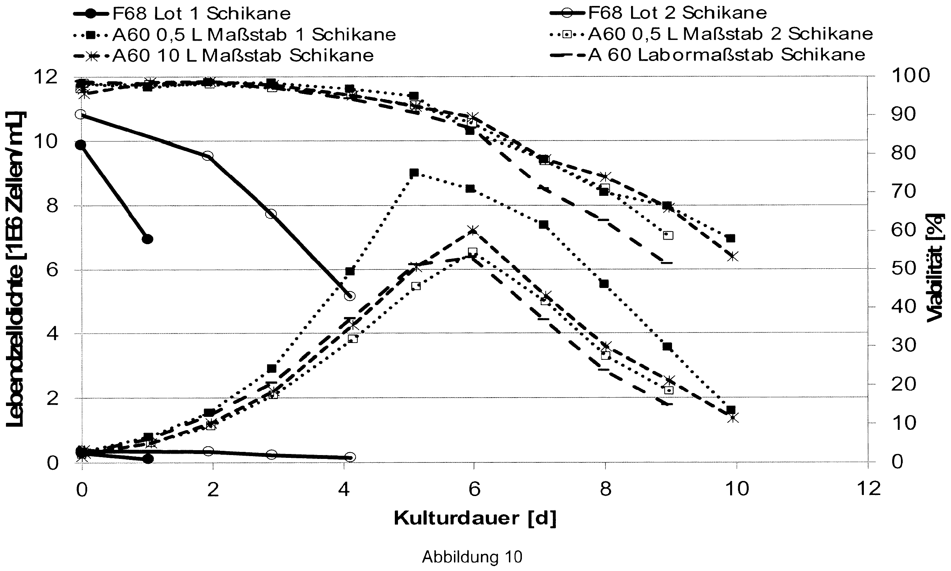

In einem weiteren Ansatz wurden HEK F Zellen in HEK TF Medium (serum und protein frei, ohne Pluronic^{®} F68) (Bestellnr. 861 (Xell AG, Bielefeld)) unter Zugabe der verschiedenen Lots von P(EtOx) im Vergleich zu zwei Lots des Pluronic^{®} F68 kultiviert, um mögliche Effekte der Hochskalierung des Produktionsprozesses erkennen zu können. Um auch hier wiederum die protektive Eigenschaft gegenüber erhöhten Scherkräften zu untersuchen, wurde diese Testung sowohl in Schüttelkolben ohne Schikane als auch mit Schikane durchgeführt. Die Ergebnisse dieser Testung sind in in den Abbildungen 9 und 10 dargestellt.

Abbildung 9 zeigt Wachstumskurve und Viabilitätsverlauf der HEK F Zellen in HEK TF unter Zugabe verschiedener Lots an P(EtOx) aus der Hochskalierung des Produktionsprozesses in Schüttelkolben ohne Schikane.

Abbildung 9 zeigt die Ergebnisse in Schüttelkolben ohne Schikane. Mit Ausnahme der Pluronic Lot 1 Kultur (7,8 ·10⁶ Zellen/mL) zeigten alle Kulturen vergleichbare maximale Lebendzelldichten von 9-10,1·10⁶ Zellen/mL und es konnte kein bedeutender Unterschied der verschiedenen P(EtOx) Lots untereinander erkannt werden.

Abbildung 10 zeigt Wachstumskurve und Viabilitätsverlauf der HEK F Zellen in HEK TF unter Zugabe verschiedener Lots an P(EtOx) aus der Hochskalierung des Produktionsprozesses in Schüttelkolben mit Schikane.

Die Kultivierungen in Schüttelkolben mit Schikane zeigten wiederum, dass Zellen unter Zugabe von P(EtOx) eine höhere Schertoleranz aufweisen als solche mit Pluronic^{®} F68 als Surfactant; die Kulturen mit Pluronic^{®} F68 zeigten bereits nach 24 h Viabilitätseinbrüche um 20 % (Lot 1) bzw. 10 % (Lot 2). Die Kulturen mit verschiedenen P(EtOx)-Lots erreichten maximale Lebendzelldichten von 6,4-9·10⁶ Zellen/mL, wobei ein Lot aus dem 0,5 L Produktionsmaßstab im Vergleich zu den übrigen Lots ein höheres Wachstum zeigte. Unter erhöhtem Scherstress zeigten sich somit nur leichte Unterschiede der verschiedenen P(EtOx) Lots aus der Hochskalierung des Produktionsprozesses, welche unter Bedingungen ohne erhöhten Scherstress in dieser Testung nicht erkennbar waren.

### Beispiel 6: Transfektionseffizienz

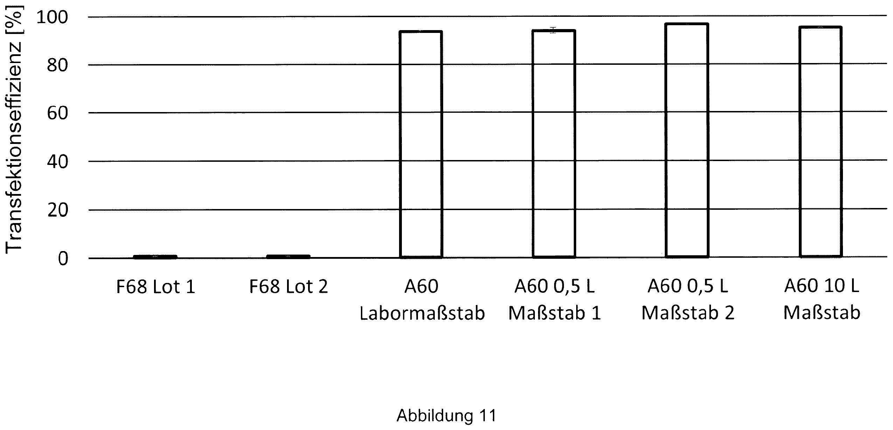

Zur Überprüfung der Transfektionseffizienz wurden zudem jeweils zwei Schüttelröhrchen der Kulturen aus den Schüttelkolben ohne Schikane angelegt und die HEK F Zellen mittels Polyethylenimin (PEI) mit einem GFP-Plasmid transfiziert. 48 Stunden nach Transfektion wurden Zelldichten, Viabilitäten und die Transfektionseffizienz dieser Kulturen aufgenommen. Die Ergebnisse dieser Messungen sind in den Abbildungen 11 bis 13 dargestellt.

Abbildung 11 zeigt Mittelwerte der Transfektionseffizienzen der HEK F Zellen in HEK TF Medium. Als Surfactant für die Kultivierungen im Schüttelröhrchen mit anschließender Transfektion eines GFP-Plasmids mittels PEI wurden zwei Pluronic^{®} F68 Lots sowie die verschiedenen Lots des Hochskalierens der P(EtOx)-Produktion eingesetzt.

Abbildung 11 zeigt, dass die Transfektionseffizienzen der Kulturen mit den verschiedenen P(EtOx) Lots allesamt über 90 % betrugen (93,5%-96,7%). Dabei konnten keine nennenswerten Unterschiede der Transfektionseffizienzen unter den einzelnen Skalierungsschritten festgestellt werden. Die Kulturen der beiden Pluronic^{®} F68 Lots konnten in diesem Versuch nicht erfolgreich transfiziert werden, die Effizienzen lagen bei unter 1%.

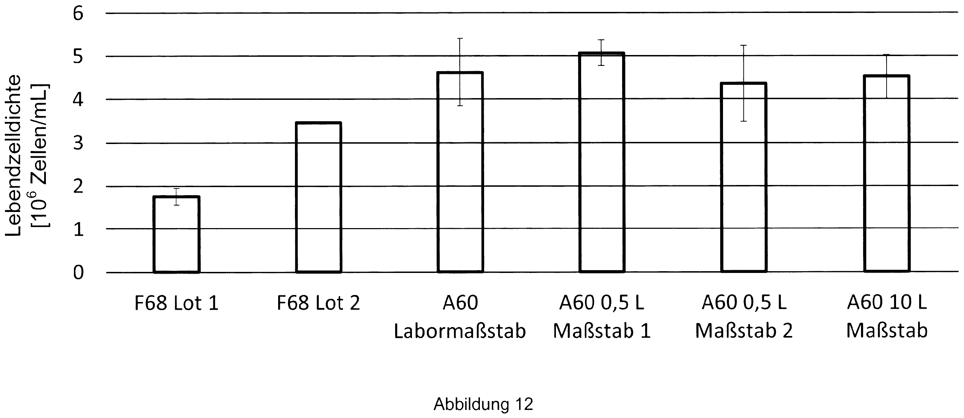

Abbildung 12 zeigt Mittelwerte der Lebendzelldichten 48 h nach Transfektion der HEK F Zellen in HEK TF Medium. Als Surfactant für die Kultivierungen im Schüttelröhrchen mit anschließender Transfektion eines GFP-Plasmids mittels PEI wurden zwei Pluronic^{®} F68 Lots sowie die verschiedenen Lots des Hochskalierens der P(EtOx)-Produktion eingesetzt.

In Abbildung 12 sind die Lebendzelldichten 48 h nach Transfektion dargestellt. Hierbei ist ersichtlich, dass sich die Mittelwerte der Lebendzelldichten der Kulturen mit den verschiedenen P(EtOx) Lots nicht nennenswert unterschieden. Die Werte variierten zwischen 4,4·10⁶ Zellen/mL und 5,1·10⁶ Zellen/mL. Die zwei Pluronic^{®} F68 Lots wiesen Lebendzelldichten von 1,8·10⁶ Zellen/mL (Lot 1) bzw. 3,5·10⁶ Zellen/mL (Lot 2) auf.

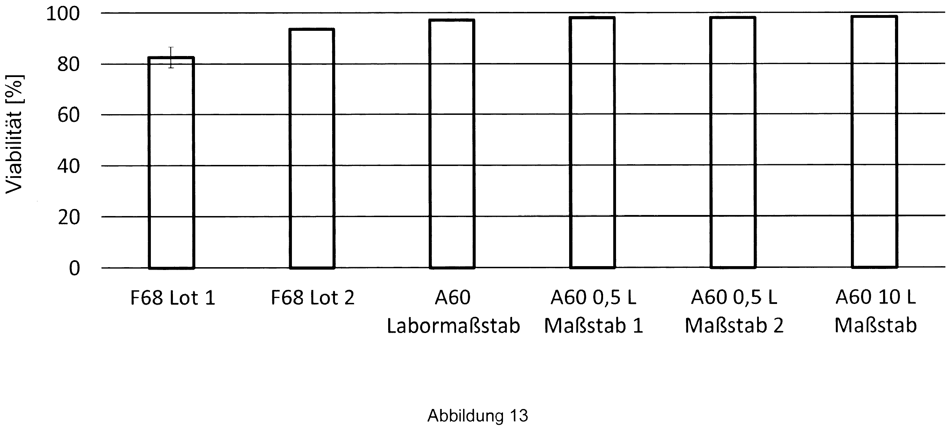

Abbildung 13 zeigt Mittelwerte der Viabilitäten 48 h nach Transfektion der HEK F Zellen in HEK TF Medium. Als Surfactant für die Kultivierungen im Schüttelröhrchen mit anschließender Transfektion eines GFP-Plasmids mittels PEI wurden zwei Pluronic^{®} F68 Lots sowie die verschiedenen Lots des Hochskalierens der P(EtOx) Produktion eingesetzt.

Die Mittelwerte der Viabilitäten der transfizierten Kulturen 48 h nach Transfektion sind Abbildung 13 zu entnehmen. Hierbei ist ersichtlich, dass die PO_{X} Kulturen eine hohe Viabilität zwischen 97,5 % und 98,2 % zeigten und kein nennenswerter Unterschied der P(EtOx) Lots untereinander erkennbar war. Die Viabilitäten der Pluronic^{®} F68 Kulturen lag bei 82,4 % (Lot 1) und 93,6 % (Lot 2).

## Patentansprüche

1. Zellkulturen enthaltend in einem turbulent bewegten Zellkulturmedium ein oder mehrere wasserlösliche Poly(oxazolin)e, mit der Maßgabe, dass das Zellkulturmedium im Wesentlichen kein Serum enthält.

2. Zellkulturen nach Anspruch 1, **dadurch gekennzeichnet, dass** das Zellkulturmedium im Wesentlichen frei von tierischen Bestandteilen ist.

3. Zellkulturen nach mindestens einem der Ansprüche 1 bis 2, **dadurch gekennzeichnet, dass** das eingesetzte Zellkulturmedium serum-frei und/oder protein-frei ist.

4. Zellkulturen nach mindestens einem der Ansprüche 1 bis 3, **dadurch gekennzeichnet, dass** das Zellkulturmedium ein chemisch definiertes Medium ist.

5. Zellkulturen nach mindestens einem der Ansprüche 1 bis 4, **dadurch gekennzeichnet, dass** diese Zellen enthalten, die im Zellkulturmedium suspendiert vorliegen.

6. Zellkulturen nach mindestens einem der Ansprüche 1 bis 5, **dadurch gekennzeichnet, dass** die Zellen suspensions-adaptierte Zellen sind.

7. Zellkulturen nach mindestens einem der Ansprüche 1 bis 6, **dadurch gekennzeichnet, dass** diese zur Proteinproduktion, Virus- und/oder Viruspartikelproduktion, Untersuchung von Stoffwechsel, Teilung und/oder weiteren zellulären Prozessen eingesetzt werden.

8. Zellkulturen nach mindestens einem der Ansprüche 1 bis 7, **dadurch gekennzeichnet, dass** diese Zelllinien ausgewählt aus mindestens einer der folgenden Gruppen enthalten: 293-T, A431, A549, BCP1, bEnd.3, BHK-21, BxPC-3, BY-2, CHO, CMT, COS-1, COS-7, CV-1, EPC, HDMEC-T, HEK, HeLa, HepG2, HL-60, HMEC-1, HUVEC-T, HT-1080, Jurkat, K562, LNCaP, MCF-7, MCF-10A, MDCK II, MDT-1A, MyEnd, Neuro-2A, NIH-3T3-T, NTERA-2 cl.D1, P19, PANC-1, Peer, RTL-W1-T, Sf-9, Saos-2, T2, T84 oder U-937.

9. Zellkulturen nach Anspruch 8, **dadurch gekennzeichnet, dass** die Zelllinien ausgewählt werden aus der Gruppe bestehend aus CHO oder HEK, insbesondere HEK 293.

10. Zellkulturen nach mindestens einem der Ansprüche 1 bis 7, **dadurch gekennzeichnet, dass** diese Hybridomazellen enthalten.

11. Zellkulturen nach mindestens einem der Ansprüche 1 bis 7, **dadurch gekennzeichnet, dass** diese Stammzellen enthalten.

12. Zellkulturen nach mindestens einem der Ansprüche 1 bis 11, **dadurch gekennzeichnet, dass** diese 0,01 bis 15 Gew. %, vorzugsweise 0,075 bis 15 Gew. %, mehr bevorzugt 0,05 bis 10 Gew. %, äußerst bevorzugt 0,1 bis 10 Gew. % an wasserlöslichen Poly(oxazolin)en, bezogen auf die Gesamtmenge der Zellkultur, enthalten.

13. Zellkulturen nach mindestens einem der Ansprüche 1 bis 12, **dadurch gekennzeichnet, dass** das wasserlösliche Poly(oxazolin) mindestens 80 Gew.-%, bezogen auf dessen Gesamtmasse, an wiederkehrenden Struktureinheiten der Formel I und/oder der Formel II aufweist
-NR¹-CH₂-CH₂- (I),
-NR¹-CH₂-CH₂-CH₂- (II),
worin
R¹ einen Rest der Formel -CO-R² bedeutet,
R² ausgewählt wird aus der Gruppe bestehend aus Wasserstoff, Methyl, Ethyl, -CₘH₂ₘ-X oder -(CₙH₂ₙ-O)ₒ-(CₚH₂ₚ-O)_{q}-R³,
R³ Wasserstoff oder C₁-C₆-Alkyl ist,
m eine ganze Zahl von 1 bis 6 ist,
X ausgewählt wird aus der Gruppe bestehend aus Hydroxyl, Alkoxy, Amino, N-Alkylamino, N, N-Dialkylamino, Carboxyl, Carbonsäureester, Sulfonyl, Sulfonsäureester oder Carbamat,
n und p unabhängig voneinander ganze Zahlen von 2 bis 4 sind, wobei n ungleich p ist, und
o und q unabhängig ganze Zahlen von 0 bis 60 sind, wobei mindestens eines der o oder q ungleich 0 ist.

14. Zellkulturen nach Anspruch 13, **dadurch gekennzeichnet, dass** das wasserlösliche Poly(oxazolin) mindestens 90 Gew.-%, bezogen auf dessen Gesamtmasse, an wiederkehrenden Struktureinheiten der Formel I aufweist, worin R² Methyl oder Ethyl bedeutet.

15. Zellkulturen nach mindestens einem der Ansprüche 1 bis 14, **dadurch gekennzeichnet, dass** diese einen oder mehrere Wirkstoffe, insbesondere pharmazeutische Wirkstoffe und/oder Impfstoffe, enthalten.

16. Zellkulturen nach mindestens einem der Ansprüche 1 bis 15, **dadurch gekennzeichnet, dass** die wasserlöslichen Poly(oxazolin)e eine Molmasse im Bereich von 5.000 bis 50.000 g/mol aufweisen.

17. Zellkulturen nach mindestens einem der Ansprüche 1 bis 16, **dadurch gekennzeichnet, dass** in diesen Zellen eingesetzt sind, die keine Fibroblasten, Pankreaszellen oder Makrophagen sind.

18. Verfahren zur Proteinproduktion, Viren- und/oder Viruspartikelproduktion, Untersuchung von Stoffwechsel, Teilung und/oder weiteren zellulären Prozessen umfassend die Kultivierung von Zellkulturen nach mindestens einem der Ansprüche 1 bis 17.

19. Verfahren nach Anspruch 18, **dadurch gekennzeichnet, dass** dieses ferner die folgende Maßnahme enthält:
Befüllen eines Bioreaktors mit Zellen und einem Zellkulturmedium gemäß einem der Ansprüche 1 bis 17.

20. Verfahren nach Anspruch 18 oder 19, **dadurch gekennzeichnet, dass** die Dauer der Kultivierung der Zellen bei satzweisem Verfahren 5 Stunden bis 30 Tage, vorzugsweise 5 Stunden bis 21 Tage und insbesondere 10 Stunden bis 15 Tage beträgt und bei kontinuierlichen Verfahren betragen 10 Tage bis 180 Tage, vorzugsweise 10 Tage bis 60 Tage und insbesondere 15 Tage bis 35 Tage beträgt.

21. Verfahren nach mindestens einem der Ansprüche 18 bis 20, **dadurch gekennzeichnet, dass** der Inhalt des Bioreaktors gerührt wird.

22. Verfahren nach mindestens einem der Ansprüche 18 bis 21, **dadurch gekennzeichnet, dass** der Bioreaktor zur Erzeugung von Turbulenzen Stromstörer enthält.

23. Verwendung von wasserlöslichen Poly(oxazolin)en zur Stabilisierung von Zellkulturen mit turbulent bewegtem Zellkulturmedium.

24. Verwendung nach Anspruch 23, **dadurch gekennzeichnet, dass** die wasserlöslichen Poly(oxazolin)e in ein Zellkulturmedium gemäß einem der Ansprüche 1 bis 4 eingebracht werden.

25. Verwendung nach Anspruch 23 oder 24, **dadurch gekennzeichnet dass** die Zellkultur 0,01 bis 15 Gew. %, vorzugsweise 0,075 bis 15 Gew. %, mehr bevorzugt 0,05 bis 10 Gew. %, und äußerst bevorzugt 0,1 bis 10 Gew. % an wasserlöslichen Poly(oxazolin)en, bezogen auf die Gesamtmenge der Zellkultur, enthält.

26. Verwendung nach mindestens einem der Ansprüche 23 bis 25, **dadurch gekennzeichnet, dass** das wasserlösliche Poly(oxazolin) mindestens 80 Gew.-%, bezogen auf dessen Gesamtmasse, an wiederkehrenden Struktureinheiten der Formel I und/oder der Formel II aufweist
-NR¹-CH₂-CH₂- (I),
-NR¹-CH₂-CH₂-CH₂- (II),
worin
R¹ einen Rest der Formel -CO-R² bedeutet,
R² ausgewählt wird aus der Gruppe bestehend aus Wasserstoff, Methyl, Ethyl, -CₘH₂ₘ-X oder -(CₙH₂ₙ-O)ₒ-(CₚH₂ₚ-O)_{q}-R³,
R³ Wasserstoff oder C₁-C₆-Alkyl ist,
m eine ganze Zahl von 1 bis 6 ist,
X ausgewählt wird aus der Gruppe bestehend aus Hydroxyl, Alkoxy, Amino, N-Alkylamino, N, N-Dialkylamino, Carboxyl, Carbonsäureester, Sulfonyl, Sulfonsäureester oder Carbamat,
n und p unabhängig voneinander ganze Zahlen von 2 bis 4 sind, wobei n ungleich p ist, und
o und q unabhängig ganze Zahlen von 0 bis 60 sind, wobei mindestens eines der o oder q ungleich 0 ist.

27. Verwendung nach Anspruch 26, **dadurch gekennzeichnet, dass** das wasserlösliche Poly(oxazolin) mindestens 90 Gew.-%, bezogen auf dessen Gesamtmasse, an wiederkehrenden Struktureinheiten der Formel I aufweist, worin R² Methyl oder Ethyl bedeutet.

## Claims

1. Cell cultures containing in a turbulently agitated cell culture medium one or more water-soluble poly(oxazoline)s, with the proviso that the cell culture medium contains substantially no serum.

2. Cell cultures according to claim 1, **characterized in that** the cell culture medium is substantially free of animal components.

3. Cell cultures according to at least one of claims 1 to 2, **characterized in that** the cell culture medium used is serum-free and/or protein-free.

4. Cell cultures according to at least one of claims 1 to 3, **characterized in that** the cell culture medium is a chemically defined medium.

5. Cell cultures according to at least one of claims 1 to 4, **characterized in that** these contain cells which are suspended in the cell culture medium.

6. Cell cultures according to at least one of claims 1 to 5, **characterized in that** the cells are suspension-adapted cells.

7. Cell cultures according to at least one of claims 1 to 6, **characterized in that** these are used for protein production, virus and/or virus particle production, investigation of metabolism, division and/or further cellular processes.

8. Cell cultures according to at least one of claims 1 to 7, **characterized in that** these contain cell lines which are selected from at least one of the following groups: 293-T, A431, A549, BCP1, bEnd.3, BHK-21, BxPC-3, BY-2, CHO, CMT, COS-1, COS-7, CV-1, EPC, HDMEC-T, HEK, HeLa, HepG2, HL-60, HMEC-1, HUVEC-T, HT-1080, Jurkat, K562, LNCaP, MCF-7, MCF-10A, MDCK II, MDT-1A, MyEnd, Neuro-2A, NIH-3T3-T, NTERA-2 cl.D1, P19, PANC-1, Peer, RTL-W1-T, Sf-9, Saos-2, T2, T84 or U-937.

9. Cell cultures according to claim 8, **characterized in that** the cell lines are selected from the group consisting of CHO or HEK, in particular HEK 293.

10. Cell cultures according to at least one of claims 1 to 7, **characterized in that** these contain hybridoma cells.

11. Cell cultures according to at least one of claims 1 to 7, **characterized in that** these contain stem cells.

12. Cell cultures according to at least one of claims 1 to 11, **characterized in that** these contain 0.01 to 15 % by weight, preferably 0.075 to 15 % by weight, more preferred 0.05 to 10 % by weight, still more preferred 0.1 to 10 % by weight of water-soluble poly(oxazoline)s, based on the total amount of the cell culture.

13. Cell cultures according to at least one of claims 1 to 12, **characterized in that** the water-soluble poly(oxazoline) comprises at least 80% by weight, based on its total mass, of recurring structural units of the formula I and/or of the formula II
-NR¹-CH₂-CH₂- (I),
-NR¹-CH₂-CH₂-CH₂- (II),
wherein
R¹ represents a residue of formula -CO-R²,
R² is selected from the group consisting of hydrogen, methyl, ethyl, -CₘH₂ₘ-X or -(CₙH₂ₙ-O)ₒ-(CₚH₂ₚ-O)_{q}-R³,
R³ is hydrogen or C₁-C₆-alkyl,
m is an integer from 1 to 6,
X is selected from the group consisting of hydroxyl, alkoxy, amino, N-alkylamino, N, N-dialkylamino, carboxyl, carboxylic acid ester, sulfonyl, sulfonic acid ester or carbamate,
n and p independently of one another are integers from 2 to 4, where n is not equal to p, and
o and q independently of one another are integers from 0 to 60, wherein at least one of o or q is not equal to 0.

14. Cell cultures according to claim 13, **characterized in that** the water-soluble poly(oxazoline) contains at least 90% by weight, based on its total mass, of recurring structural units of the formula I, wherein R² is methyl or ethyl.

15. Cell cultures according to at least one of claims 1 to 14, **characterized in that** these contain one or more active substances, in particular pharmaceutical ingredients and/or vaccines.

16. Cell cultures according to at least one of claims 1 to 15, **characterized in that** the water-soluble poly(oxazoline)s have a molar mass in the range from 5,000 to 50,000 g/mol.

17. Cell cultures according to at least one of claims 1 to 16, **characterized in that** these are used in cells other than fibroblasts, pancreatic cells or macrophages.

18. A method for protein production, virus and/or virus particle production, investigation of metabolism, division and/or further cellular processes comprising the cultivation of cell cultures according to at least one of claims 1 to 17.

19. Method according to claim 18, **characterized in that** this further comprises the following measure:
filling a bioreactor with cells and a cell culture medium according to any one of claims 1 to 17.

20. Method according to claim 18 or 19, **characterized in that** the duration of the cultivation of the cells in batchwise processes is between 5 hours and 30 days, preferably between 5 hours and 21 days and in particular between 10 hours and 15 days and in continuous processes is between 10 days and 180 days, preferably between 10 days and 60 days and in particular between 15 days and 35 days.

21. Method according to at least one of claims 18 to 20, **characterized in that** the contents of the bioreactor are stirred.

22. Method according to at least one of claims 18 to 21, **characterized in that** the bioreactor contains baffles to generate turbulence.

23. Use of water-soluble poly(oxazoline)s for stabilizing cell cultures with turbulently agitated cell culture medium.

24. Use according to claim 23, **characterized in that** the water-soluble poly(oxazoline)s are introduced into a cell culture medium according to any one of claims 1 to 4.

25. Use according to claim 23 or 24, **characterized in that** the cell culture contains 0.01 to 15% by weight, preferably 0.075 to 15% by weight, more preferred 0.05 to 10% by weight, and still more preferred 0.1 to 10% by weight of water-soluble poly(oxazolin)s, based on the total amount of the cell culture.

26. Use according to at least one of claims 23 to 25, **characterized in that** the water-soluble poly(oxazoline) comprises at least 80% by weight, based on its total mass, of recurring structural units of the formula I and/or of the formula II
-NR¹-CH₂-CH₂- (I),
-NR¹-CH₂-CH₂-CH₂- (II),
wherein
R¹ represents a residue of formula -CO-R²,
R² is selected from the group consisting of hydrogen, methyl, ethyl, -CₘH₂ₘ-X or -(CₙH₂ₙ-O)ₒ-(CₚH₂ₚ-O)_{q}-R³,
R³ is hydrogen or C₁-C₆-alkyl,
m is an integer from 1 to 6,
X is selected from the group consisting of hydroxyl, alkoxy, amino, N-alkylamino, N, N-dialkylamino, carboxyl, carboxylic acid ester, sulfonyl, sulfonic acid ester or carbamate,
n and p independently of one another are integers from 2 to 4, where n is not equal to p, and
o and q independently of one another are integers from 0 to 60, wherein at least one of o or q is not equal to 0.

27. Use according to claim 26, **characterized in that** the water-soluble poly(oxazoline) comprises at least 90% by weight, based on its total mass, of recurring structural units of the formula I, wherein R² is methyl or ethyl.

## Revendications

1. Cultures cellulaires contenant une ou plusieurs poly(oxazoline)(s) hydrosoluble(s) au sein d'un milieu de culture cellulaire agité de manière turbulente, sous réserve que le milieu de culture cellulaire ne contienne essentiellement pas de sérum.

2. Cultures cellulaires selon la revendication 1, **caractérisées en ce que** le milieu de culture cellulaire est essentiellement exempt de matières animales.

3. Cultures cellulaires selon au moins l'une quelconque des revendications 1 à 2, **caractérisées en ce que** le milieu de culture cellulaire utilisé est exempt de sérum et/ou exempt de protéines.

4. Cultures cellulaires selon au moins l'une quelconque des revendications 1 à 3, **caractérisées en ce que** le milieu de culture cellulaire est un milieu chimiquement défini.

5. Cultures cellulaires selon au moins l'une quelconque des revendications 1 à 4, **caractérisées en ce que** lesdites cultures cellulaires contiennent des cellules qui sont en suspension dans le milieu de culture cellulaire.

6. Cultures cellulaires selon au moins l'une quelconque des revendications 1 à 5, **caractérisées en ce que** les cellules sont des cellules adaptées à la suspension.

7. Cultures cellulaires selon au moins l'une quelconque des revendications 1 à 6, **caractérisées en ce que** lesdites cultures cellulaires sont utilisées pour la production de protéines, la production de virus et/ou de particules virales, l'étude du métabolisme, de la division et/ou d'autres processus cellulaires.

8. Cultures cellulaires selon au moins l'une quelconque des revendications 1 à 7, **caractérisées en ce que** lesdites cultures cellulaires contiennent des lignées cellulaires choisies parmi au moins l'un des groupes ci-dessous : 293-T, A431, A549, BCP1, bEnd.3, BHK-21, BxPC-3, BY-2, CHO, CMT, COS-1, COS-7, CV-1, EPC, HDMEC-T, HEK, HeLa, HepG2, HL-60, HMEC-1, HUVEC-T, HT-1080, Jurkat, K562, LNCaP, MCF-7, MCF-10A, MDCK II, MDT-1A, MyEnd, Neuro-2A, NIH-3T3-T, NTERA-2 cl.D1, P19, PANC-1, Peer, RTL-W1-T, Sf-9, Saos-2, T2, T84 ou U-937.

9. Cultures cellulaires selon la revendication 8, **caractérisées en ce que** les lignées cellulaires sont choisies parmi le groupe constitué de CHO ou HEK, en particulier HEK 293.

10. Cultures cellulaires selon au moins l'une quelconque des revendications 1 à 7, **caractérisées en ce que** lesdites cultures cellulaires contiennent des cellules d'hybridomes.

11. Cultures cellulaires selon au moins l'une quelconque des revendications 1 à 7, **caractérisées en ce que** lesdites cultures cellulaires contiennent des cellules souches.

12. Cultures cellulaires selon au moins l'une quelconque des revendications 1 à 11, **caractérisées en ce que** lesdites cultures cellulaires contiennent entre 0,01 et 15 % en poids, de manière préférée entre 0,075 et 15 % en poids, de manière plus préférée entre 0,05 et 10 % en poids, de la manière la plus préférée entre 0,1 et 10 % en poids de poly(oxazoline)s hydrosolubles, par rapport à la quantité totale de culture cellulaire.

13. Cultures cellulaires selon au moins l'une quelconque des revendications 1 à 12, **caractérisées en ce que** la poly(oxazoline) hydrosoluble présente au moins 80 % en poids, par rapport à sa masse totale, d'unités structurelles récurrentes de formule I et/ou de formule II
-NR¹-CH₂-CH₂- (I),
-NR¹-CH₂-CH₂-CH₂- (II),
où
R¹ représente un radical de formule -CO-R²,
R² est choisi parmi le groupe constitué des radicaux hydrogène, méthyle, éthyle, - CₘH₂ₘ-X ou -(CₙH₂ₙ-O)ₒ-(CₚH₂ₚ-O)_{q}-R³,
R³ est un atome d'hydrogène ou un radical alkyle en C₁-C₆,
m est un entier de 1 à 6,
X est choisi parmi le groupe constitué des radicaux hydroxyle, alcoxy, amino, N-alkylamino, N, N-dialkylamino, carboxyle, ester d'acide carboxylique, sulfonyle, ester d'acide sulfonique ou carbamate,
n et p sont, indépendamment l'un de l'autre, des nombres entiers de 2 à 4, n étant différent de p, et
o et q sont, indépendamment l'un de l'autre, des nombres entiers de 0 à 60, au moins un parmi o ou q n'étant pas égal à 0.

14. Cultures cellulaires selon la revendication 13, **caractérisées en ce que** la poly(oxazoline) hydrosoluble présente au moins 90 % en poids, par rapport à sa masse totale, d'unités structurelles récurrentes de formule I, R² représentant un radical méthyle ou éthyle.

15. Cultures cellulaires selon au moins l'une quelconque des revendications 1 à 14, **caractérisées en ce que** lesdites cultures cellulaires contiennent un ou plusieurs principe(s) actif(s), en particulier des principes actifs pharmaceutiques et/ou des vaccins.

16. Cultures cellulaires selon au moins l'une quelconque des revendications 1 à 15, **caractérisées en ce que** les poly(oxazoline)s hydrosolubles présentent une masse molaire située dans la plage comprise entre 5 000 et 50 000 g/mole.

17. Cultures cellulaires selon au moins l'une quelconque des revendications 1 à 16, **caractérisées en ce que** des cellules qui ne sont pas des fibroblastes, des cellules pancréatiques ou des macrophages sont utilisées au sein desdites cultures cellulaires.

18. Procédé de production de protéines, de production de virus et/ou de particules virales, d'étude du métabolisme, de la division et/ou d'autres processus cellulaires, comprenant la mise en culture de cultures cellulaires selon au moins l'une quelconque des revendications 1 à 17.

19. Procédé selon la revendication 18, **caractérisé en ce que** ledit procédé comprend en outre l'étape ci-dessous consistant à :
remplir un bioréacteur avec des cellules et un milieu de culture cellulaire selon l'une quelconque des revendications 1 à 17.

20. Procédé selon la revendication 18 ou 19, **caractérisé en ce que** la durée de la culture des cellules est comprise entre 5 heures et 30 jours, de manière préférée entre 5 heures et 21 jours et de manière particulièrement préférée entre 10 heures et 15 jours dans le cas d'un procédé séquentiel et est comprise entre 10 jours et 180 jours, de manière préférée entre 10 jours et 60 jours et de manière particulièrement préférée entre 15 jours et 35 jours dans le cas d'un procédé continu.

21. Procédé selon l'une quelconque des revendications 18 à 20, **caractérisé en ce que** le contenu du bioréacteur est agité.

22. Procédé selon au moins l'une quelconque des revendications 18 à 21, **caractérisé en ce que** le bioréacteur contient des perturbateurs de courant afin de générer des turbulences.

23. Utilisation de poly(oxazoline)s hydrosolubles pour stabiliser des cultures cellulaires avec un milieu de culture cellulaire agité de manière turbulente.

24. Utilisation selon la revendication 23, **caractérisée en ce que** les poly(oxazoline)s hydrosolubles sont introduites dans un milieu de culture cellulaire selon l'une quelconque des revendications 1 à 4.

25. Utilisation selon la revendication 23 ou 24, **caractérisée en ce que** la culture cellulaire contient entre 0,01 et 15 % en poids, de manière préférée entre 0,075 et 15 % en poids, de manière plus préférée entre 0,05 et 10 % en poids, et de la manière la plus préférée entre 0,1 et 10 % en poids % de poly(oxazoline)s hydrosolubles, par rapport à la quantité totale de culture cellulaire.

26. Utilisation selon au moins l'une quelconque des revendications 23 à 25, **caractérisée en ce que** la poly(oxazoline) hydrosoluble présente au moins 80 % en poids, par rapport à sa masse totale, d'unités structurelles récurrentes de formule I et/ou de formule II
-NR¹-CH₂-CH₂- (I),
-NR¹-CH₂-CH₂-CH₂- (II),
où
R¹ représente un radical de formule -CO-R²,
R² est choisi parmi le groupe constitué des radicaux hydrogène, méthyle, éthyle, - (CₘH₂ₘ-X ou -(CₙH₂ₙ-O)ₒ-(CₚH₂ₚ-O)_{q}-R³,
R³ est un atome d'hydrogène ou un radical alkyle en C₁-C₆,
m est un entier de 1 à 6,
X est choisi parmi le groupe constitué des radicaux hydroxyle, alcoxy, amino, N-alkylamino, N, N-dialkylamino, carboxyle, ester d'acide carboxylique, sulfonyle, ester d'acide sulfonique ou carbamate,
n et p sont, indépendamment l'un de l'autre, des nombres entiers de 2 à 4, n étant différent de p, et
o et q sont, indépendamment l'un de l'autre, des nombres entiers de 0 à 60, au moins un parmi o ou q n'étant pas égal à 0.

27. Utilisation selon la revendication 26, **caractérisée en ce que** la poly(oxazoline) hydrosoluble présente au moins 90 % en poids, par rapport à sa masse totale, d'unités structurelles récurrentes de formule I, R² représentant un radical méthyle ou éthyle.
